# EUROPEAN PATENT SPECIFICATION

(11) **EP 3 755 381 B1**
(45) Date of publication and mention of the grant of the patent: **01.01.2025**
(21) Application number: 19705208.7
(22) Date of filing: 21.02.2019
(51) Int. Cl.: A61K 49/00

(54) **OPTICAL IMAGING AGENTS TARGETING INFLAMMATION**
AUF ENTZÜNDUNGEN GERICHTETE, OPTISCHE KONTRASTMITTEL
AGENTS D'IMAGERIE OPTIQUE CIBLANT L'INFLAMMATION

(30) Priority: 21.02.2018 EP 18305183
(43) Date of publication of application: 30.12.2020
(73) Proprietor: Sorbonne Université, 75006 Paris (FR); Institut National de la Santé et de la Recherche Médicale (INSERM), 75013 Paris (FR); Ecole Nationale Superieure de Chimie de Paris, 75005 Paris (FR); Centre National de la Recherche Scientifique (CNRS), 75016 Paris (FR); Université Paris Cité, 75006 Paris (FR)
(72) Inventor: ZHANG, Yongmin, 92160 ANTONY (FR); BESSODES, Michel, 94800 VILLEJUIF (FR); SEGUIN, Johanne, 94270 KREMLIN BICETRE (FR); MIGNET, Nathalie, 92140 CLAMART (FR); SCHERMAN, Daniel, 75012 PARIS (FR)
(74) Representative: Cabinet Beau de Loménie
(86) International application number: PCT/EP2019/054382
(87) International publication number: WO 2019/162417

(56) References cited:
- LEMDANI K. ET AL: "Assessment of the targeting specificity of a fluorescent albumin conceived as a preclinical agent of the liver function", NANOSCALE, 22 November 2018 (2018-11-22), pages 21151 - 21160, XP093051930, Retrieved from the Internet <URL:https://pubs.rsc.org/en/content/articlepdf/2018/nr/c8nr04163f> [retrieved on 20230605], DOI: 10.1039/C8NR04163F
- JI BAK KIM ET AL: "Intravascular optical imaging of high-risk plaques in vivo by targeting macrophage mannose receptors", SCIENTIFIC REPORTS, vol. 6, no. 1, 7 March 2016 (2016-03-07), XP055488353, DOI: 10.1038/srep22608
- CHANIKARN CHANTARASRIVONG ET AL: "Synthesis and Functional Characterization of Novel Sialyl LewisX Mimic-Decorated Liposomes for E-selectin-Mediated Targeting to Inflamed Endothelial Cells", MOLECULAR PHARMACEUTICS, vol. 14, no. 5, 24 February 2017 (2017-02-24), US, pages 1528 - 1537, XP055488736, ISSN: 1543-8384, DOI: 10.1021/acs.molpharmaceut.6b00982
- MI KYUNG YU ET AL: "Targeting Strategies for Multifunctional Nanoparticles in Cancer Imaging and Therapy", THERANOSTICS, vol. 2, no. 1, 1 January 2012 (2012-01-01), AU, pages 3 - 44, XP055488580, ISSN: 1838-7640, DOI: 10.7150/thno.3463
- MAHMOUD RAZAVIAN ET AL: "Optical imaging of MMP-12 active form in inflammation and aneurysm", SCIENTIFIC REPORTS, vol. 6, no. 1, 1 December 2016 (2016-12-01), XP055488904, DOI: 10.1038/srep38345
- YANG YANG ET AL: "Fluorescent Neomannosyl Bovine Serum Albumin as Efficient Probe for Mannose Receptor Imaging and MCF-7 Cancer Cell Targeting", ACS APPLIED NANO MATERIALS, vol. 1, no. 3, 23 February 2018 (2018-02-23), pages 1058 - 1065, XP055488850, ISSN: 2574-0970, DOI: 10.1021/acsanm.7b00134

## Description

### Field of the invention

The present invention concerns optical imaging agents targeting inflammation biomarkers. The optical imaging agents of the invention are in particular useful in the *in vivo* as well as *ex vivo* diagnosis of diseases and conditions associated with inflammatory diseases such as cancer, renal failure, stroke.

### Background of the invention

Recently, optical imaging has drawn increasing interest because of the ease of use of portable imaging devices, and reduced toxicity and risk to the operator and patient compared in particular with X-ray imaging, radiomedicine or magnetic resonance imaging.

Optical imaging, and more specifically fluorescence imaging, are used for analysis of biological samples (such as biopsies for instance, hereafter referred to as *"ex vivo* imaging"), in 2 dimensions (2D) as well as 3 dimensions (3D) (for instance using tomography), and can be used *in vivo* during surgery, for instance using portable devices such as those commercialized by Fluoptics and Novadaq.

Optical imaging requires administering optical agents to the patient or animal to be imaged (*in vivo* or *ex vivo*) prior to imaging. For instance, Kim et. al. (Scientific Reports, vol. 6, No. 1, 22608, (07.03.2016)) describes an optical imaging probe, comprising: a glycol chitosan nanoparticle with attached cyanine fluorophore and maleimide-PEG-mannose targeting inflammation.

However, to improve the quality and accuracy of diagnostic using optical imaging, there remains a need for optical imaging agents able to target specific markers in the body. In particular, there is a need for optical imaging agents selectively targeting markers of inflammation, in order to diagnose inflammatory diseases *in vitro* or *ex vivo* or *in vivo.* Such optical imaging agents would thus enable the diagnostic of cancer or inflammatory diseases, and would be useful for image-guided surgery, for instance during tumour resection.

### Summary of the invention

The present invention thus concerns an optical imaging agent of formula (SIGNAL)ₙ-SUPPORT-(L-BIOVECTOR)ₘ, wherein:
SUPPORT represents a physiologically acceptable chemical or biological substrate, with a particle size comprised between 1 and 100 nm,
SIGNAL is a fluorophore as defined in claim 1,
L is a linker of formula -C(X)-R¹-Y-, with X being O, NH, or S
R¹ being a (C₁-C₆)alkyl group, preferably a (C₃-C₅)alkyl group, optionally interrupted by 1 to 3 groups selected from a heteroatom such as O or NH, a -C(O)- group, a -NHC(O)- group, a -(O)CNH- group, -C(O)-NH-N=C- group, a -N=C- group, a -NH-SO₂-NH-C(S)- group and a 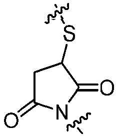 group ,
Y being NH or a -(O)CNH- group, and
BIOVECTOR is a carbohydrate able to target markers of inflammation as defined in claim 1, a carbohydrate able to target markers of inflammation as defined in claim 1,
wherein the mean grafting value n is greater than or equal to 0.5 and is less than 3,
wherein the mean grafting value m is between 0 and 30, preferably between 1 and 30,

Signal is selected from the group consisting of fluorophores, preferentially fluorophores emitting in the near Infra-red, such as indocyanin green, cyanin-5, cyanin 5,5, cyanin-7, alexa fluors, and derivatives thereof,
a pharmaceutically acceptable salt, solvate or hydrate thereof.

The optical imaging agents of the invention thus exhibit several advantages.

First, the SIGNAL and BIOVECTOR parts of the optical imaging agent of the invention are covalently linked to its SUPPORT part - through the linker L in the case of BIOVECTOR, when present. Such covalent linkage allows for a much-improved selectivity of the optical imaging agent of the invention for inflamed tissues, as compared with agents wherein the BIOVECTOR or the SIGNAL parts are linked to the rest of the imaging agent only through ionic, hydrogen or Van der Waals bonds. Indeed, in the latter case, dissociation of the different parts of the imaging agent could occur in the biological tissues or the body, thus shading doubt as to the accuracy of the observed images. Second, the optical imaging agent of the invention selectively targets markers of inflammation, thus allowing for accurate delimitation of inflamed areas of the body using *in vivo* or *ex vivo* optical imaging, in particular fluorescence imaging. Thanks to the portable imaging devices available nowadays, such optical imaging agent can thus be used as a real-time aid to surgery: for instance, after resection of a tumour, it is possible to visualize through optical imaging of the area of interest if all of the tumoral tissues have been removed, or if a further resection is necessary. Other imaging techniques are either more complex to use in this context (in particular MRI, scintigraphy, PET), or less sensitive (echography).

Third, the presence of a SUPPORT linking the SIGNAL part of the optical imaging agent of the invention and its BIOVECTOR part, increases the bioavailability of the optical imaging agent of the invention.

Finally, it is noteworthy that the optical imaging agent of the invention comprises up to 3 fluorophores. Indeed, the inventors have surprisingly demonstrated that the presence of more than 3 fluorophores have deleterious effect on the intensity of the observed signal, in contrast for instance to what is commonly known for magnetic resonance or scintigraphy imaging agents (see for instance WO 2008/074960).

According to another aspect, the present invention relates to a diagnostic composition comprising at least one optical imaging agent of formula (SIGNAL)ₙ-SUPPORT-(L-BIOVECTOR)ₘ of the invention, a pharmaceutically acceptable salt, solvate or hydrate thereof, and at least one pharmaceutically acceptable excipient as defined in claim 5.

According to another aspect, the present invention relates to the optical imaging agent of formula (SIGNAL)ₙ-SUPPORT-(L-BIOVECTOR)ₘ of the invention or the diagnostic composition of the invention for in vivo use as a diagnostic agent, in particular for diagnosing diseases or conditions associated with inflammation.

According to another aspect, the present invention relates to a method of imaging a biological tissue *ex vivo,* using optical or fluorescence imaging, said biological tissue comprising the optical imaging agent or the diagnostic composition of the invention.

According to another aspect, the present invention relates to a method of *ex vivo* diagnosing a disease or condition associated with inflammation, comprising *ex vivo* imaging a biological tissue of a patient in need thereof, in particular a biological tissue obtained through biopsy, said biological tissue comprising the optical imaging agent or the diagnostic composition of the invention.

### Detailed description of the invention

### Optical Imaging Agent

n and m are chosen so as to avoid any destabilization of SUPPORT. Indeed, the optical imaging agent of the invention should be able to form stable colloidal suspensions in physiologically acceptable solutions.

As used herein, a "stable suspension" is understood as a suspension which does not form any aggregates or does not sediment over a period of at least 6 hours, preferably at least 12 hours, even more preferably at least 24 hours.

As used herein, a "physiologically acceptable solution" is understood as a solution that is nontoxic and suitable for administration to a patient via oral route or parenteral route, in particular by injection, without any undesired side-effects. As such, the osmolarity and osmolality of a physiologically acceptable solution should be controlled so as to avoid any undesired side effects. Examples of solutes suitable for forming a physiologically acceptable solution are water (in particular water for injection, saline, and aqueous solutions such as NaCl 150mM, PBS, glucose 5%, Hepes 20mM + glucose 5%, lactose 5%, Hepes 20mM + lactose 5%).

In the present invention, a "patient" is understood as an animal, preferably a mammal such as a rodent (mice or rats), and preferably a human being.

Numbers n and m are calculated with mass spectrometry experiments. It is known from the skilled person in the art that mass spectrometry characterization is used to assess grafting of fragments on macromolecules.

After the grafting of the SIGNAL and/or the BIOVECTOR fragment, a mass spectrometry analysis provides the additional mass of the grafted molecule compared to the mass of the native SUPPORT. The mass difference allows attribution of a mean grafting value regarding the number of SIGNAL and/or BIOVECTOR fragments present on the molecule.

n is greater than or equal to 0.5 and is less than 3, for instance n is between 0.5 and 2.5. n is between 0.5 and 2. n can also be greater than or equal to 1 and is less than 3, for instance n is between 1 and 2, preferably n is equal to 1 or 2. According to the present invention the mean grafting value n is greater than or equal to 0.5 and is less than 2. m can be between 0 and 30, preferably between 1 and 30, more preferably between 5 and 20. According to the present invention the mean grafting value m is between 1 and 30.

In a particular embodiment, n is between 0.5 and 2, and m is between 1 and 30, more preferably between 5 and 20.

### SUPPORT

In general formula (I), SUPPORT represents a physiologically acceptable chemical or biological substrate, that is to say an entity whose administration is compatible with a living being. Typically, SUPPORT is conjugated to SIGNAL and L-BIOVECTOR through all or part of its amino (NH₂) groups located on its surface.

Advantageously, SUPPORT has a particle size of between 1 and 100 nm, preferably between 5 and 50, more preferably of between 5 nm and 20 nm. In a particular embodiment, SUPPORT has a particle size of between 5 and 10 nm.

SUPPORT may itself have biological activity, or on the contrary be totally inert. Advantageously, the SUPPORT is biodegradable.

As used herein, a "biodegradable" entity is an entity which is biodegraded *in vivo* through the action of proteins such as enzymes, but which however does not degrade spontaneously in a physiological solution in particular at a pH of between 6 and 8 and at a temperature of between 25°C and 37°C, generally for a time period of at most 7 days. SUPPORT is preferably selected from proteins. Suitable proteins or protein conjugates are for instance described in WO 2004/071536 (see in particular section "Nature of the carrier material" pages 15-18).

As used herein, "protein" is meant as a macromolecule composed of a string (or sequence) of amino acids linked together by peptide bonds. Proteins suitable for the present invention may have various molecular weights, in particular ranging from 10 to 500 kDa, preferably from 50 to 100 kDa. They may be chosen from albumin, ovalbumin, lactalbumin, immunoglobulins, macroglobulins, microglobulins, lipoproteins, circulating hormones and factors, hemocyanins, and derivatives thereof, preferably albumin, in particular a human albumin such as human serum albumin (HSA).

In a preferred embodiment, SUPPORT is a protein, especially albumin, preferably human serum albumin (HSA) or derivatives thereof, with a particle size of between 2 and 60, nm preferably of between 5 and 20 nm. In particular, use may be made of albumins commercialized by Vialebex or Baxter. Such a small particle size, combined with the fact that optical agent does not aggregate, is of particular interest in terms of biodistribution and bioavailability. According to this embodiment, the optical imaging agent in which SUPPORT is albumin has a size of between 6 nm and 20 nm.

Albumin derivatives are in particular ovalbumin and lactalbumin.

In another embodiment, SUPPORT may also be selected from polysaccharides, nanoparticles (including liposomes), microparticles (including liposomes) or biocompatible polymers.

In the context of the present invention, a polymer is characterized as "biocompatible" if the polymer and its degradation products are non-toxic to the animal or human being to which it is administered, and does not induce adverse effects in the host's body, e.g. immune reaction at the injection site. Biocompatible polymers suitable for the present invention may be chosen from any of the polymers known to those skilled in the art, including poly(N-(2-hydroxypropyl)methacrylamide) (HMPA), polyethylene glycol (PEG), collagen, polysaccharides, poly(2-methoxyethylacrylate) (PMEA), polydimethylsiloxane (PDMS), polyvinyl pyrrolidone (PVP), hyaluronic acid (HA), heparan, heparanesulfates, dextrans, dextransulfates, heparins, cyclodextrins and derivatives thereof.

When SUPPORT is a nano- and microparticle, said particle may in particular be made of biodegradable organosoluble polymer.

As used herein, "particles" are understood as particles with an average particle size of between 1 and 6 000 nm, and in particular between 2 and 1000 nm, preferably between 3 and 100 nm, and in particular between 3 and 30 nm. The particles of the invention have a particle size of between 1 and 100 nm and typically have an organic core (such as a polymeric core), but may also be liposomes. More specifically, "microparticles" are understood as particles with an average particle size of between 500 nm and 6 000 nm, while "nanoparticles" are understood as particles with an average particle size of between 1 and 500 nm, in particular between 2 and 300 nm, preferably between 3 and 150 nm, and in particular between 5 nm and 100 nm, such as between 3 and 30 nm.

As biodegradable organosoluble polymer, mention may be made of polyesters such as poly (lactic acid) (PLA), poly (glycolic acid) (PGA), poly(ε-caprolactone) (PCL), polyanhydrides, poly (hyaluronan), poly(alkylcyanoacrylates), polyorthoesters, poly (alkylene tartrate), polyphosphazenes, polyamino acids, polyamidoamines, polysaccharides, polycarbonates, polymethylidenemalonate, polysiloxane, polyhydroxybutyrate or poly(malic acid), and copolymers thereof.

Where appropriate, the particle surface may be modified for example to confer to said particle additional properties, such as hydrophilicity, so as to improve their *in vivo* reactivity, for example by promoting their adsorption of plasma proteins.

Particularly preferred are "targeted nanoparticles", such as targeted liposomes or targeted biocompatible polymers. As used herein, a "targeted nanoparticle" is able to target markers of inflammation, such as Selectin E. In particular, a "targeted nanoparticle" has a biomarker on its surface targeting markers of inflammation. Suitable nanoparticles and microparticles are for instance described in WO 2006/116742.

Conjugates of specific-binding moieties can be used for detecting specific target molecules in biological samples. The specific-binding portion (in the present invention: BIOVECTOR) of such conjugates binds tightly to a target in the sample and the signal-generating portion (herein SIGNAL) is utilized to provide a detectable signal that indicates the presence/and or location of the target.

### SIGNAL

SIGNAL is a fluorophore, preferably an organic fluorophore, allowing the optical agent of the invention to be detected through optical imaging, in particular fluorescence imaging. In particular, SIGNAL is a hydrophilic fluorophore. Of note, the fluorophore is hydrophilic enough to avoid any precipitation of the optical imaging agent of the invention. SIGNAL is a hydrophilic cyanine derivative, which are well-known fluorophores.

According to the invention, SIGNAL is of formula (Ia) below: 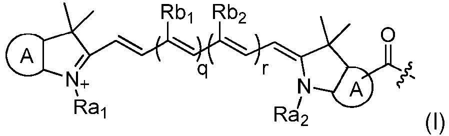 wherein
q is 0 or 1,
r is 0 or 1,
R_{b1} and R_{b2} are H or a C₁-C₄ group, or are bridged to form a -CH₂-CH₂-CH₂-alkylene group (only when q and r represent 1),
Rₐ₁ and Rₐ₂ are identical or different and are independently a (C₁-C₆)alkyl group, optionally substituted by a SO₃⁻ group, a SO₃Na or a SO₃K group, or a COOH group, provided that not more than one of Rₐ₁ and Rₐ₂ is substituted by a SO₃⁻ group.

Ring A represents a C₅-C₆ monocyclic aryl or heteroaryl group or a C₈-C₁₂ fused bicyclic aryl or heteroaryl group, preferably a C₅-C₆ monocyclic aryl group or a C₈-C₁₂ fused bicyclic aryl group, such as a phenyl or naphthyl group, and Ring A is optionally substituted by one SO₃⁻, SO₃Na or SO₃K group, provided that not more than one of Rₐ₁ and Rₐ₂ is substituted by a SO₃⁻ group, provided that the compound of formula (I) comprises not more than one SO₃⁻ group. The compound of formula (I) may have an overall electric charge, namely a positive charge. In such case, it is provided as a salt, in particular a halogenide salt such as a chloride Cl⁻ salt.

The compound of formula (I) is preferably hydrophilic, and as such advantageously contains at least one SO₃⁻ or SO₃Na group, preferably it contains one SO₃⁻ group and SO₃Na group.

In a particular embodiment, SIGNAL is of formula (Ia) below: 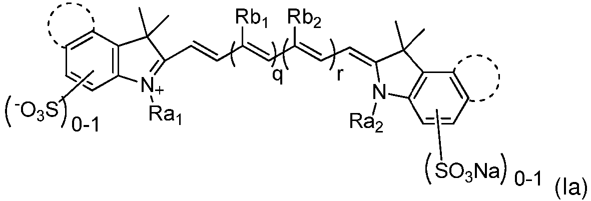
wherein q, r, Rₐ₁, Rₐ₂, R_{b1} and R_{b2} are as defined above or below, and Ring 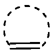 is optional, and when present represents a (C₅-C₆) monocyclic aryl or heteroaryl group (fused with the adjacent indoline group) optionally substituted by one SO₃⁻ or SO₃Na group, provided that not more than one of Rₐ₁ and Rₐ₂ is substituted by a SO₃⁻ group, preferably a (C₅-C₆) monocyclic aryl group such as phenyl optionally substituted by one SO₃⁻ , SO₃Na or SO₃K group,
provided that the compound of formula (I) comprises not more than one SO₃⁻ group.

In a particular embodiment, SIGNAL is of formula (Ia1) below: 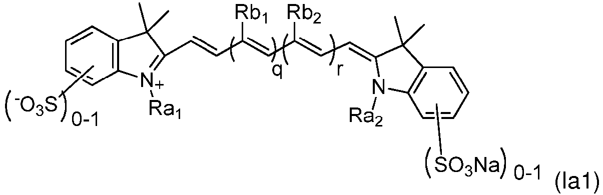 wherein q, r, Rₐ₁, Rₐ₂, R_{b1} and R_{b2} are as defined above or below.

In another particular embodiment, SIGNAL is of formula (la2) below: 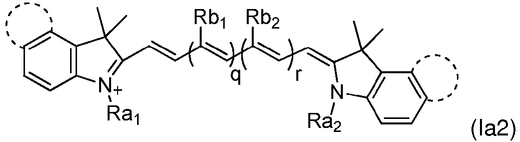 wherein q, r, Rₐ₁, Rₐ₂, R_{b1} and R_{b2} are as defined above or below.

In a particular embodiment of formulae (I), (Ia), (la1) and (la2):
R_{b1} and R_{b2} are advantageously H;
q is 0 or 1 and advantageously r is 0;
Rₐ₁ is preferably methyl
Rₐ₂ is preferably -CH₂-CH₂-CH₂-CH₂-CH₂-COOH.

In another particular embodiment, of formulae (I), (Ia), (la1) and (la2):
R_{b1} and R_{b2} are advantageously H;
q and r are both 1, and R_{b1} and R_{b2} taken together represent a -CH₂-CH₂-CH₂- alkylene group;
Rₐ₁ is preferably methyl
Rₐ₂ is preferably -CH₂-CH₂-CH₂-CH₂-CH₂-COOH.

In another particular embodiment, SIGNAL is of formula (lb) below: 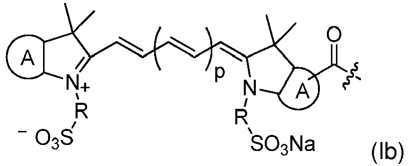 wherein
p is an integer of between 1 and 4, preferably between 1 and 2,
R is a (C₁-C₆)alkyl group, preferably a (C₁-C₄)alkyl group,
Ring A represents a C₅-C₆ monocyclic aryl or heteroaryl group or a C₈-C₁₂ fused bicyclic aryl or heteroaryl group, preferably a C₅-C₆ monocyclic aryl group or a C₈-C₁₂ fused bicyclic aryl group, such as a phenyl or naphthyl group.

In the compound of formula (I), each ring A is fused with the adjacent pyrrolidine group. Advantageously, SIGNAL is of formula (Ib1) below: 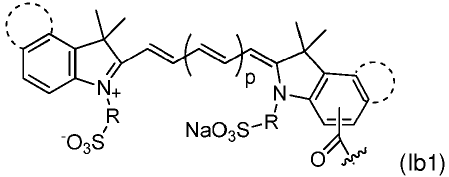 wherein
p is an integer of between 1 and 2,
R is a (C₁-C₄)alkyl group
Ring 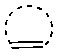 is optional, and when present represents a (C₅-C₆) monocyclic aryl or heteroaryl group (fused with the adjacent indoline group), preferably a (C₅-C₆) monocyclic aryl group such as phenyl.

In a particular embodiment, SIGNAL is of formula (Ib2) below: 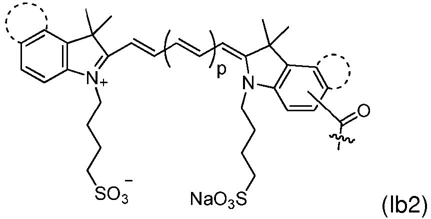 wherein
p is an integer of between 1 and 2, and
Ring 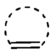 is optional, and when present represents a (C₅-C₆) monocyclic aryl or heteroaryl group (fused with the adjacent indoline group), preferably a (C₅-C₆) monocyclic aryl group, such as phenyl.

In a preferred embodiment, SIGNAL is selected from cyanines and indocyanines of formulae below: 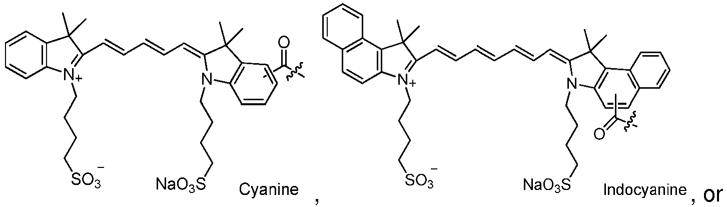 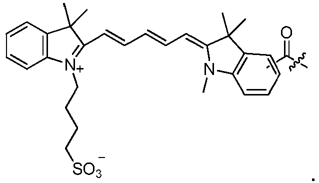 more specifically, SIGNAL is

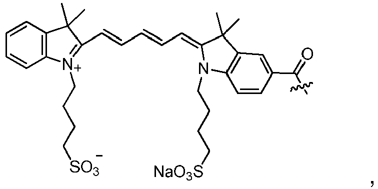

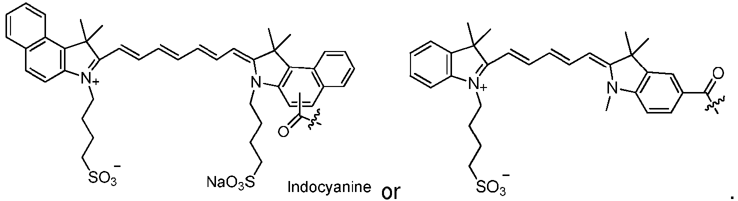

In another preferred embodiment, SIGNAL is selected from: 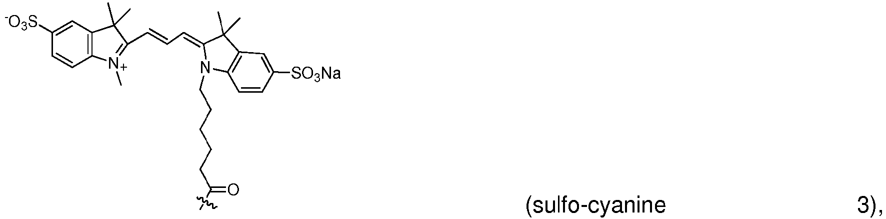 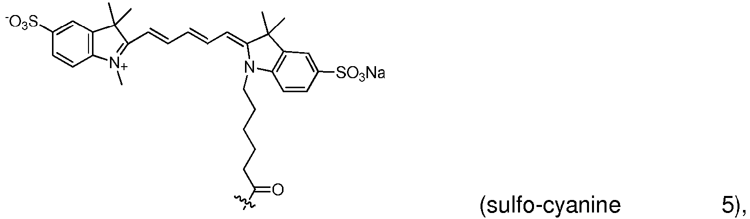 and

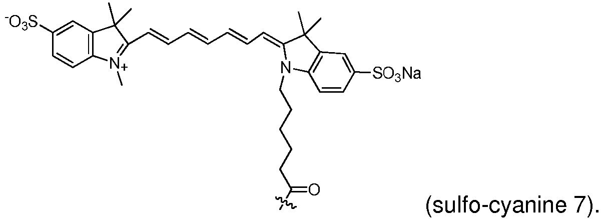

Of note, when excited with light (in particular at a wavelength of between 450 nm and 1000 nm, preferably in the "close red domain" or "close infrared domain" i.e. between 600 nm and 900 nm), cyanine emits red light, whereas indocyanine emits green light, through fluorescence phenomena.

The above fluorophores of formula (I) are known in the art and are commercially available, in particular from Luminoprobe.

### BIOVECTOR

The BIOVECTOR is a carbohydrate as defined in claim 1 targeting selectively a marker of inflammation.

The term "carbohydrate" as used in the present disclosure refers to a monosaccharide or polysaccharide, or nitrogen-derivatives thereof. Monosaccharides are in particular erythrose, threose, ribose, arabinose, xylose, lyxose, allose, altrose, glucose, mannose, fucose, gulose, idose, galactose, talose, erythrulose, ribulose, xylulose, psicose, fructose, sorbose, tagatose. The carbohydrate of the invention is preferably not protected (i.e. it does not contain any O-protecting groups). However, the carbohydrate of the invention may contain one or two sulfate groups, *i.e.* a OSO₃H or preferably a OSO₃Na group in lieu of a OH group.

Polysaccharides are preferably bi-, tri- or quadrisaccharides, and each monosaccharide group of which it is composed is preferably selected from the monosaccharides listed above.

Nitrogen derivatives of monosaccharides and polysaccharides are understood herein as compounds having a monosaccharide or polysaccharide structure, wherein 1 to 2 oxygen atoms in a monosaccharide or 1 to 4 oxygen atoms in a polysaccharide are replaced with a NH group.

Carbohydrates targeting selectively a marker of inflammation are known to the person of skill in the art. In particular, lactose is known not to belong to this category. A typical example is Lewis^{x} pentasaccharide molecule in which the Lewis^{x} trisaccharide is linked to a lactose molecule, however the Lewis^{X}-Lewis^{X} interaction is due to the Lewis^{x} trisaccharide, but not to lactose.

Preferred carbohydrates targeting selectively a marker of inflammation are mannose, glucose, fucose, sialyl Lewis^{x} (or SLe^{X}), or any mono- or polysaccharide comprised in sialyl Lewis^{x}, such as neuraminic acid or and the trisaccharide of formula: 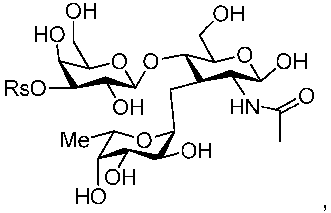 with Rs being H, SO₃Na or SO₃K, preferably H or
SO₃Na,
and derivatives thereof.

Examples of carbohydrate derivatives are carbohydrates wherein one anomeric OH group is replaced by a O-(C₁-C₆)alkyl-X' group, with X' representing O, NH or S, preferably NH. For instance a carbohydrate derivatives is a carbohydrate wherein an anomeric OH group is replaced by a -O-CH₂-CH₂-NH group. When the carbohydrate comprises a COOH group, a particular carbohydrate derivative is a carbohydrate wherein the group COOH is replaced by a group of formula COO-(C₁-C₆)alkyl-X', such as a COOCH₂CH₂NH group.

In an advantageous embodiment, BIOVECTOR is selected from the group consisting of mannose and sialyl Lewis^{x} (or SLe^{x}), and derivatives thereof.

Sialyl Lewis^{x} (or SLe^{x}) is indeed an efficient ligand for E-selectin, also known as CD62 antigen-like family member E (CD62E), endothelial-leukocyte adhesion molecule 1 (ELAM-1), or leukocyte-endothelial cell adhesion molecule 2 (LECAM2). E-selectin is an adhesion molecule expressed only on endothelial cells activated by cytokines. During inflammation, E-selectin plays an important part in recruiting leukocytes to the site of injury: local release of cytokines IL-1 and TNF-α by damaged cells induces the over-expression of E-selectin on endothelial cells of nearby blood vessels. Leukocytes in the blood expressing the correct ligand will then bind with low affinity to E-selectin. As such, E-selectin is a particularly useful marker of inflammation, in particular overexpressed in the direct vicinity of tumour metastases.

In this context, BIOVECTOR is preferably of formula (II): 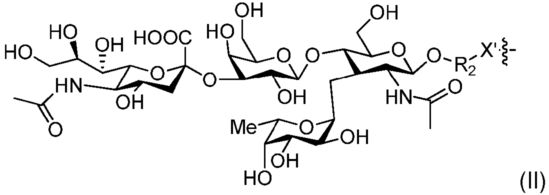
wherein R² represents a (C₁-C₆)alkyl group, preferably a (C₁-C₄)alkyl group,
and X' represents a heteroatom such as O, S or NH, preferably NH.

In particular, BIOVECTOR is :

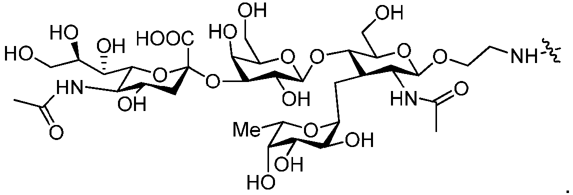

This particular BIOVECTOR may be obtained from the corresponding compound described for instance described in Lu et al. (Carbohydrate Research 2014, 383, 89-96). Substructures of Sialyl Lewis X are also useful ligand for E-selectin such as neuraminic acid. In this context, BIOVECTOR is advantageously of formula (III): 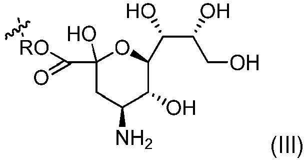 wherein R is R₂-X', with R² and X' as defined above in connection with formula (II).

Also considered is a trisaccharides of formula (IV): 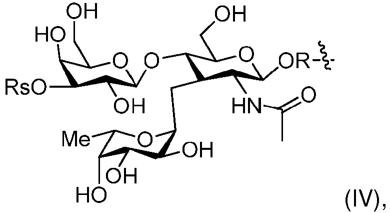 wherein Rs is H or SO₃Na, and R is as defined above in connection with formula (III).

Mannose is also an efficient ligand for macrophages, overexpressed at a site of inflammation, which is useful as a marker of inflammation. A preferred mannose derivative is a compound of formula (V): 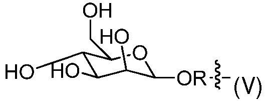 wherein R is as defined above in connection with formula (III).

Fucose is also a known to be associated with inflammatory conditions, in particular in cancer. In this context, BIOVECTOR is advantageously of formula (VI): 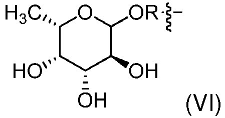 wherein R is as defined above in connection with formula (III)

Because of the link between diabetes and inflammation in particular, glucose is associated with markers of inflammation. In this context, BIOVECTOR is advantageously of formula (VII), or (VII'): 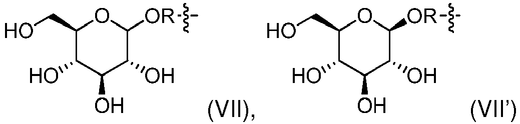 wherein R is as defined above in connection with formula (III).

### Linker L

In a particular embodiment, L is a linker of formula -C(X)-R¹-Y-, wherein
X is O or NH
R¹ is a (C₁-C₆)alkyl, preferably a (C₃-C₅)alkyl, optionally interrupted by 1 to 3 (preferably 1) groups selected from a heteroatom such as O or NH, a -C(O)- group, a - NHC(O)- group, a -(O)CNH- group, and a 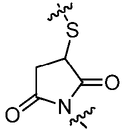 group ,
Y is NH or a -(O)CNH- group.

Advantageously, X is NH.

Preferably, R¹ is a (C₁-C₆)alkyl, preferably a (C₃-C₅)alkyl, optionally interrupted by a 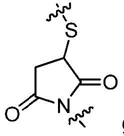 group.

### Particular embodiments

In a preferred embodiment, the optical imaging agent of the invention is of formula (SIGNAL)ₙ-SUPPORT-(L-BIOVECTOR)ₘ, wherein:
SUPPORT is a protein, especially an albumin such as human serum albumin (HSA) or derivatives thereof, with a particle size of between 1 and 100 nm, preferably of between 5 and 50, more preferably of between 5 nm and 20 nm. In a particular embodiment, SUPPORT has a particle size of between 5 and 10 nm
SIGNAL is of formula (Ia1), (la2), (Ib) as defined above:
   Wherein, preferably,
   p is an integer of between 1 and 4, preferably between 1 and 2,
   R is a (C₁-C₆) alkyl group, preferably a (C₁-C₄) alkyl group,
   Ring A represents a (C₅-C₆) monocyclic aryl or heteroaryl group or a (C₈-C₁₂) fused bicyclic aryl or heteroaryl group, preferably a (C₅-C₆) monocyclic aryl group or a (C₈-C₁₂) fused bicyclic aryl group such as phenyl or naphthyl,
L is a linker of formula -C(X)-R¹-Y-, with
   X being O or NH
   R¹ being a (C₁-C₆)alkyl, preferably a (C₃-C₅)alkyl, optionally interrupted by 1 to 3 groups selected from a heteroatom such as O or NH, a -C(O)- group, a -NHC(O)-group, a -(O)CNH- group, and a 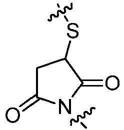 group ,
   Y being NH or a -(O)CNH- group, and

BIOVECTOR is a carbohydrate targeting markers of inflammation selected from the group consisting of mannose, glucose, fucose, neuraminic acid, sialyl Lewis^{x} (SLe^{x}), the trisaccharide :
and derivatives thereof, said carbohydrate being preferably a compound of formula (II) or (V) as defined above,
n is greater than or equal to 0.5 and is less than 2, and is preferably between 0.5 and 1.5,
m is between 1 and 30, preferably between 5 and 20.

In this preferred embodiment, SIGNAL is in particular of formula (Ib), (lb1) or (Ib2), wherein, preferably,
p is an integer of between 1 and 4, preferably between 1 and 2,
R is a (C₁-C₆) alkyl group, preferably a (C₁-C₄) alkyl group,
Ring A represents a (C₅-C₆) monocyclic aryl or heteroaryl group or a (C₈-C₁₂) fused bicyclic aryl or heteroaryl group, preferably a (C₅-C₆) monocyclic aryl group or a (C₈-C₁₂) fused bicyclic aryl group such as phenyl or naphthyl.

Even more preferably, SIGNAL is indocyanine or sulfo-cyanine 5.

In this preferred embodiment, L is advantageously a linker of formula -C(X)-R¹-Y-, with X being NH, R¹ being a (C₁-C₆)alkyl, preferably a (C₃-C₅)alkyl, optionally interrupted by 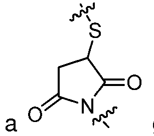 group, and Y being NH or a -(O)CNH- group.

In a preferred embodiment, the optical imaging agent of the invention is of formula (SIGNAL)ₙ-SUPPORT-(L-BIOVECTOR)ₘ, wherein:
SUPPORT is human serum albumin (HSA),
SIGNAL is 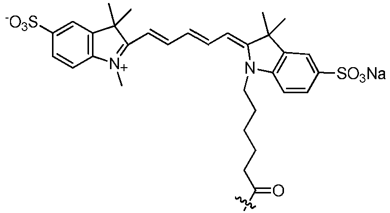 or 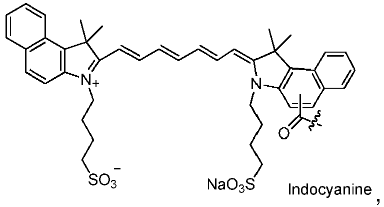
L is a linker of formula -C(X)-R¹-Y-, with
   X being O
   R¹ being a (C₃-C₅)alkyl, optionally interrupted by 1 to 3 groups selected from a heteroatom such as O or NH, a -C(O)- group, a -NHC(O)- group, a -(O)CNH-group, and a 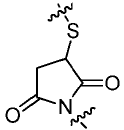 group ,
   Y being NH or a -(O)CNH- group,
n is between 0.5 and 2, and m is between 1 and 30, more preferably between 5 and 20.

### Pharmaceutical and Diagnostic Composition

The present invention also relates to a pharmaceutical or diagnostic composition comprising at least one optical imaging agent of formula (SIGNAL)ₙ-SUPPORT-(L-BIOVECTOR)ₘ as defined in the claims, a pharmaceutically acceptable salt, solvate or hydrate thereof, and at least one pharmaceutically acceptable excipient.

The pharmaceutical or diagnostic compositions of the invention are advantageously suitable for administration via oral, sublingual, subcutaneous, intramuscular, intravenous, transdermal, topical or rectal route, more preferably via oral route or by injection. The optical imaging agent of the invention can be administered in unit forms for administration, mixed with conventional pharmaceutical carriers, to animals or to humans.

When a solid composition is prepared in the form of tablets, the optical imaging agent of the invention is mixed with a pharmaceutical vehicle and other conventional excipients known to those skilled in the art.

When a composition is formulated for injection, the optical imaging agent of the invention is mixed with a saline solution and other conventional excipients known to those skilled in the art.

The optical imaging agents of the invention can be used in a diagnostic composition at a dose ranging from 0.01 mg/kg to 1000 mg/kg, for instance between 1 mg/kg and 800 mg/kg, in particular between 5 mg/kg and 500 mg/kg, preferably administered in only one dose. However, it may be necessary to use doses outside these ranges, which will then be designed by the person skilled in the art.

### Optical Imaging

In the optical imaging agent of the invention, BIOVECTOR is a carbohydrate targeting selectively a marker of inflammation, preferably mannose, sialyl Lewis^{x} (or SLe^{x}) or derivatives thereof, advantageously the derivatives of formula (II) and (III) as defined above.

Therefore, the optical imaging agents of the invention will selectively accumulate in tissues or body areas wherein the targeted markers of inflammation (E-selectin for sialyl Lewis^{x} (or SLe^{x}) derivatives, macrophages for mannose derivatives) are overexpressed. The present invention thus also relates to the optical imaging agent of formula (SIGNAL)ₙ-SUPPORT-(L-BIOVECTOR)ₘ as defined in the claims or the pharmaceutical or diagnostic composition of the invention for in vivo ise as a diagnostic agent, in particular for diagnosing diseases or conditions associated with inflammation.

The optical imaging agents or diagnostic compositions of the invention are also useful in diagnosing a cardiovascular or inflammatory disease such as myocardial ischemia stroke, inflammatory bowel disease (such as Crohn disease and ulcerative colitis), renal failure, post-operative ileus, brain ischemia, diabetes, diabetic nephropathy, metabolic syndrome, sickle-cell disease, neurodegenerative diseases such as Alzheimer's disease or Parkinson's disease, neuropathic pain, hypertension, pulmonary arterial hypertension, septicemia, septic or endotoxic shock, hemorrhagic shock, multiple sclerosis, cancer and chronic obstructive pulmonary disease, and arthritic diseases (such as arthritis and osteoarthritis).

Preferably, the optical imaging agent or diagnostic composition of the invention is used for diagnosing cancer (in particular tumours, such as tumours of colorectal cancer), stroke and renal failure.

In a particular embodiment, the disease or condition associated with inflammation is cancer, and in particular tumours, such as tumours of colorectal cancer. In this embodiment, the optical imaging agent of formula (SIGNAL)ₙ-SUPPORT-(L-BIOVECTOR)ₘ as defined by the claims or the pharmaceutical or diagnostic composition of the invention is useful as an aid to surgery, in particular as contrast agent in image-guided surgery. In this embodiment, the optical imaging agent or pharmaceutical or diagnostic composition of the invention is used in particular for real time diagnosis, allowing to follow evolution of the resection of tumoral tissues.

In this embodiment, the patient undergoing surgery is a living animal, in particular a human patient, suffering from cancer (such as colorectal cancer), and is in need of surgery for tumour resection. The optical imaging agent or diagnostic composition of the invention is administered to the patient advantageously prior to surgery or during surgery, for instance by injection.

Therefore, the present invention also relates to the optical imaging agent or diagnostic composition of the invention for use in a method of surgically resecting tumours of a patient comprising the following successive steps:
- resecting the tumoral tissue identified prior to surgery in an area of interest;
- imaging the area of interest of the patient, to whom an effective amount of the optical imaging agent or diagnostic composition of the invention has been administered prior to surgery or during surgery (for instance by injection), using an optical imaging device (in particular a fluorescence imaging device), so as to identify remaining tumoral tissues;
- when light (or fluorescence) signals are observed in the area of interest defining remaining tumoral tissues, proceeding with the resection of the identified remaining tumoral tissues; when no light (or fluorescent) signal is observed in the area of interest, not proceeding with any further resection in the area of interest.
As used herein, an "area of interest" is understood as an area of tissues or an organ of the patient comprising tumoral tissue to be resected and the vicinity of the tumoral tissue. For instance, in the case of liver cancer, the area of interest may be the whole liver. In the case of colorectal cancer, the area of interest is preferably the colon and/or the rectum, or part of it.

In this particular embodiment, the optical imaging agent or diagnostic composition of the invention thus proves useful in helping resecting the entirety of the tumoral tissues. Such a method using the optical imaging agent or diagnostic composition of the invention as an aid to surgery also allows for resecting the minimum sane tissues, whereas nowadays, in the absence of such method, surgeons tend to resect a wide area of sane tissues around the tumours as a safety measure.

The present disclosure further relates to, but is not part of the present invention, the use of the optical imaging agent of the invention, a pharmaceutically acceptable salt, solvate or hydrate thereof, for the manufacture of a diagnostic composition, in particular for use for diagnosing diseases or conditions associated with inflammation as detailed above.

In a particular embodiment, the disease or condition associated with inflammation is cancer, and in particular tumours, such as tumours of colorectal cancer. In this embodiment, the disclosure concerns the use of the optical imaging agent of the invention, a pharmaceutically acceptable salt, solvate or hydrate thereof, for the manufacture of a diagnostic composition for real time diagnosis during surgery, in particular tumour resection.

The present disclosure further concerns a method for diagnosing diseases or conditions associated with inflammation as detailed above, comprising administering an effective amount of the optical imaging agent of the invention, a pharmaceutically acceptable salt, solvate or hydrate thereof, or the diagnostic composition of the invention, to a patient in need thereof.

In a particular instance, the disease or condition associated with inflammation is cancer, and in particular tumours, such as tumours of colorectal cancer. In this instance, the method of the invention is for real time diagnosis during surgery, in particular tumour resection.

The method of the disclosure (not being part of the present invention) further relates to a method of surgically resecting tumours comprising the following successive steps:
- resecting the tumoral tissue identified prior to surgery;
- imaging the area of interest of the patient (area in the vicinity of the resected tumour(s)) to whom an effective amount of the optical imaging agent or diagnostic composition of the invention has been administered prior to surgery or during surgery (for instance by injection), using an optical imaging device (in particular a fluorescence imaging device), so as to identify tumoral tissues which would not have been resected;
- when light (or fluorescence) signals are observed in the area of interest defining remaining tumoral tissues, proceeding with the resection of the identified remaining tumoral tissues; when no light (or fluorescent) signal is observed in the area of interest, not proceeding with any further resection in the area of interest.

### Method of imaging (ex vivo)

The present invention concerns the optical imaging agents as described in the claims for image-guided surgery. The principles of image-guided surgery are for instance described in Lim et al (J Visc Surg. 2014 Apr;151(2):117-24. doi: 10.1016/j.jviscsurg.2013.11.003. Epub 2014 Jan 21. *"Indocyanine green fluorescence imaging in the surgical management of liver cancers: current facts and future implications').*

The present invention further concerns the optical imaging agents as described in the claims for in vivo use in a method for diagnosing an inflammation, in particular non-invasive optical imaging.

The present invention further concerns a method of imaging a biological tissue *ex vivo,* using optical or fluorescence imaging, said biological tissue comprising the optical imaging agent or the diagnostic composition of the invention.

The biological tissue is preferably a biopsy sample.

In a first embodiment, the patient is first administered with (an effective amount of) the optical imaging agent of the invention or the diagnostic composition of the invention, and the biopsy is then carried out to take a sample of tissue, and the tissue of the biopsy is then fixed under conditions selected by the one of skill in the art so as to interfere neither with the SIGNAL part of the optical imaging agent of the invention, nor in the BIOVECTOR-Marker of inflammation recognition. In other words, the fixation step of the tissue does not degrade it, so that imaging of the tissue faithfully reflects the state of the remaining tissue in the body.

In this first embodiment, the biopsy tissue is then imaged using an optical imaging device (more specifically a fluorescence imaging device). Devices allowing for 2D or 3D imaging may be used, depending on the thickness of the biopsy, and the condition or disease associated with inflammation from which the patient is suffering.

Examples of 3D optical imaging techniques are for example optical tomography.

In a second embodiment, the patient is not administered with the optical imaging agent of the invention or the diagnostic composition of the invention prior to the biopsy. In this second embodiment, the biopsy tissue is thus subjected to the optical imaging agent of the invention or the diagnostic composition of the invention for a time sufficient to allow the optical imaging agent to bind to the targeted marker of inflammation (e.g. E-selectin in the case of BIOVECTOR of formula (II) as defined above, or macrophages in the case of BIOVECTOR of formula (III) as defined above), and then rinsed. Then, the tissue of the biopsy is then fixed under conditions selected by the one of skill in the art so as to interfere neither with the SIGNAL part of the optical imaging agent of the invention, nor in the BIOVECTOR-Marker of inflammation recognition. In other words, the fixation step of the tissue does not degrade it, so that imaging of the tissue faithfully reflects the state of the remaining tissue in the body.

In this second embodiment, the biopsy tissue is then imaged using an optical imaging device (more specifically a fluorescence imaging device). Devices allowing for 2D or 3D imaging may be used, depending on the thickness of the biopsy, and the condition or disease associated with inflammation from which the patient is suffering.

The present invention further relates to a method of diagnosing ex vivo a disease or condition associated with inflammation as detailed above, comprising *ex vivo* imaging a biological tissue of a patient in need thereof, in particular a biological tissue obtained through biopsy as explained above, said biological tissue comprising the optical imaging agent or the diagnostic composition of the invention.

### Process of preparation

The present disclosure further provides a process for preparing the optical imaging agent of formula (SIGNAL)ₙ-SUPPORT-(L-BIOVECTOR)ₘ as defined above, a pharmaceutically acceptable salt, solvate or hydrate thereof.
the process of the invention preferably comprises the following successive steps:
a) mixing SUPPORT with a succinimide derivative of SIGNAL in an aqueous (physiologically acceptable) solution such as a saline solution, preferably a phosphate buffered saline (PBS) solution, to obtain a compound of formula (SIGNAL)ₙ-SUPPORT, and
b) when m is not 0, mixing (SIGNAL)ₙ-SUPPORT with a compound of formula H-L-BIOVECTOR in an aqueous (physiologically acceptable) solution such as a saline solution, preferably a phosphate buffered saline (PBS) solution, and
c) isolating the optical imaging agent of formula (SIGNAL)ₙ-SUPPORT-(L-BIOVECTOR)ₘ.

Optionally, the process comprises a step d) of washing the optical imaging agent of formula (SIGNAL)ₙ-SUPPORT-(L-BIOVECTOR)ₘ , preferably with water or a NaCl aqueous solution.

The succinimide derivative of SIGNAL are easily accessible from the corresponding SIGNAL compound using derivatization methods well known in the art.

In the same way, compounds of formula H-L-BIOVECTOR are easily accessible to the one of skill in the art from the corresponding BIOVECTOR, using methods well known in the field of carbohydrate chemistry, especially when L is of formula-C(X)-R¹-Y-, with X being O, R¹ being a (C₃-C₅)alkyl, optionally interrupted by 1 to 3 groups selected from a heteroatom such as O or NH, a -C(O)- group, a -NHC(O)- group, a -(O)CNH- group, and a 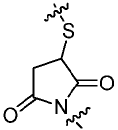 group , and Y a NH or a -(O)CNH- group.

BIOVECTOR is easily accessible from the corresponding carbohydrate, in particular when BIOVECTOR is of formula (II), (III), (V), (VI) and (VII), which may be easily prepared from Sialyl Lewis^{x}, neuraminic acid, mannose, fucose and glucose, respectively.

The compounds of formula (IV) may be easily prepared from the corresponding trisaccharide, which may be prepared as follows:

### Step 1 - preparation of an azido derivative:

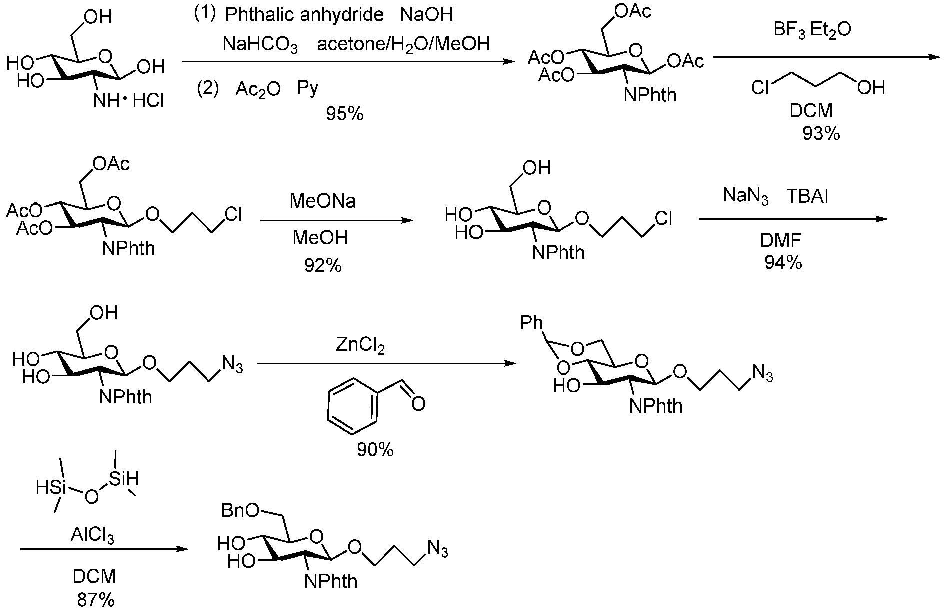

### Step 2 - preparation of a trisaccharide through carbohydrate coupling

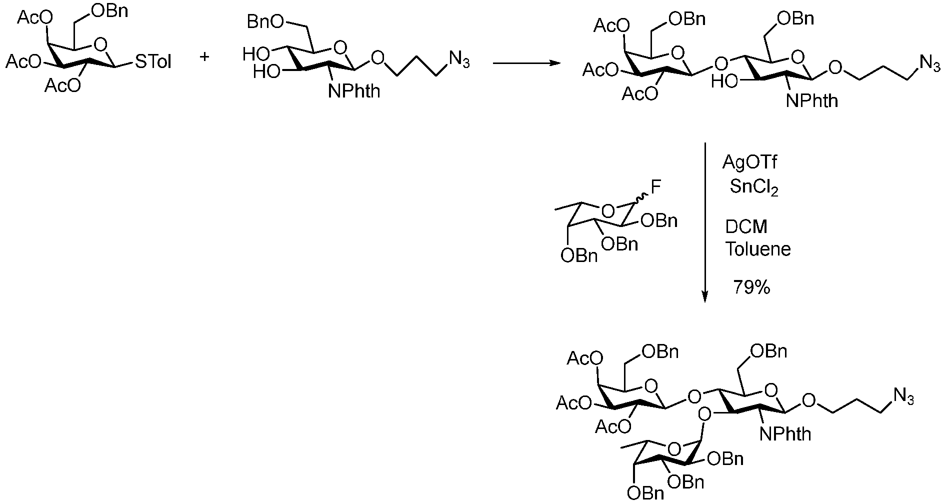

The thus obtained protected azido-trisaccharide may be deprotected as follows:

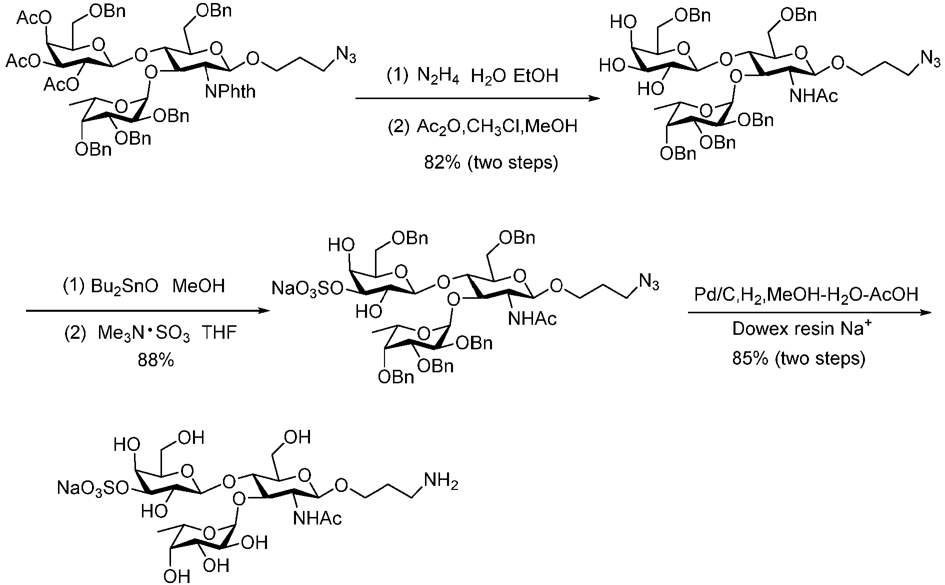

Monosaccharide 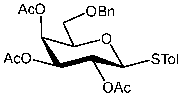 is obtained for instance as follows: 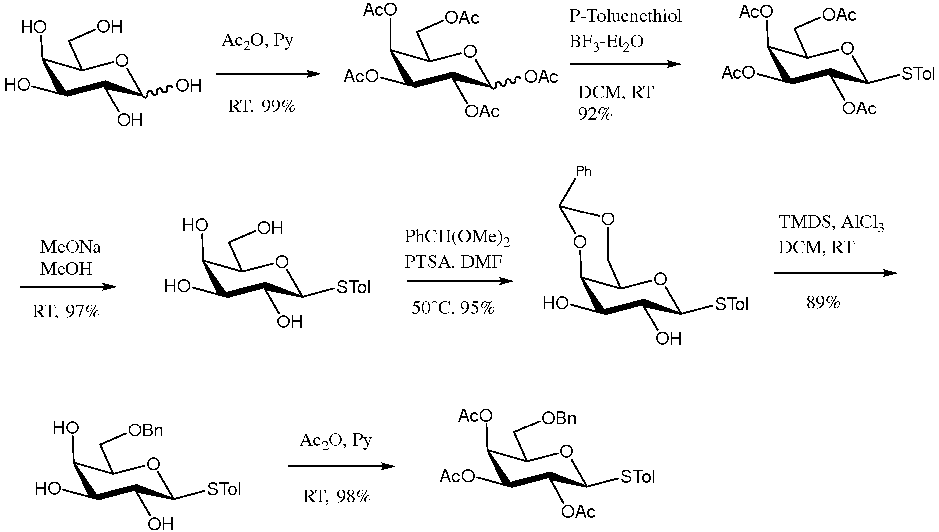 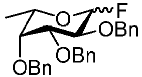 is for instance obtained as described in Lu et al. Carbohydrate Research 2014, 383, 89-96.

The compounds BIOVECTORS-LH may then for instance be prepared as follows: 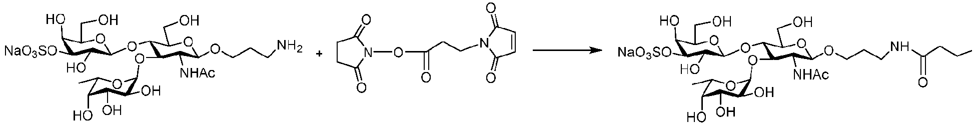 functionalization of the protein using this compound preferably occurs through a Michael addition of alpha,beta-unsaturated double bond of the maleimide moiety by a free SH group present on the surface of the protein (preferably the albumin). The free SH group is introduced on the albumin for instance by reacting albumin with the Traut's reagent (2-iminothiolane).

### Definitions

The present invention encompasses only stable compounds. In this regard, when "isomers" are referred to, only stable isomers are considered.

Within the groups, radicals or fragments defined in the description and the claims, the number of carbon atoms is specified inside the brackets. For example, (C₁-C₆)alkyl designates an alkyl group or radical having 1 to 6 carbon atoms.

In the formulas, 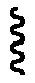 indicates the bond linked to the rest of the molecule.

As used herein, a "-(C₁-C₆)alkyl" designates an acyclic, saturated, linear or branched hydrocarbon chain comprising 1 to 6 carbon atoms. Examples of -(C₁-C₆)alkyl groups include methyl, ethyl, propyl, butyl, pentyl or hexyl. Unless explicitly stated, the definitions propyl, butyl, pentyl and hexyl include all possible isomers, in particular structural isomers. For example, butyl comprises *n*-butyl, *iso*-butyl, *sec*-butyl and *tert*-butyl.

The term "aryl" designates an aromatic, monocyclic ring that may be fused with a second saturated, unsaturated or aromatic ring. The term aryl include, without restriction to the following examples, phenyl, indanyl, indenyl, tetrahydronaphtyl and dihydronaphtyl. The most preferred monocyclic aryl is phenyl, while the most preferred bicyclic fused aryl is naphthyl. The aryl group may be substituted, preferably with one or more groups independently selected from the group consisting of alkyl, alkoxy, halogen, hydroxyl, amino, nitro, cyano, trifluoro, carboxylic acid or carboxylic ester.

The term "heteroaryl" designates aromatic, monocyclic ring that may be fused with a second saturated, unsaturated or aromatic ring where one or more carbon atoms have been replaced with one or more heteroatoms chosen from among N, O and S. Unless explicitly stated, the term "heteroaryl" includes all possible isomers, in particular position isomers. Examples of monocyclic heteroaryl groups include furyl, thienyl, imidazolyl, pyridyl, pyrrolyl, N-alkyl pyrrolyl, pyrimidinyl, pyrazinyl, tetrazolyl, triazolyl and triazinyl. Examples of fused bicyclic heteraryls include indolyl, indolinyl, benzofuryl, benthienyl, quinoleine, isoquinoleine. The heteroaryl group may be substituted, preferably with one or more groups independently selected from the group consisting of alkyl, alkoxy, halogen, hydroxyl, amino, nitro, cyano, trifluoro, carboxylic acid or carboxylic ester. Preferred heteroaryls are those having 5 or 6 atoms in the ring, such as indolyl, pyrrolyl, pyridinyl, pyrrazolyl, triazolyl, furanyl or thienyl.

For the purpose of the invention, the term "pharmaceutically acceptable" is intended to mean what is useful to the preparation of a pharmaceutical composition, and what is generally safe and non-toxic, for a pharmaceutical use.

The term « pharmaceutically acceptable salt, hydrate of solvate » is intended to mean, in the framework of the present invention, a salt of a compound which is pharmaceutically acceptable, as defined above, and which possesses the pharmacological activity of the corresponding compound. Such salts comprise:
(1) hydrates and solvates,
(2) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(3) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

In the present invention, the term "size" is understood as the hydrodynamic diameter of the particles.

In the present description, the carbohydrate Sialyl Lewis^{x} is abbreviated without any distinction to Sle^{x} or SLX.

### FIGURES

**Figure 1****:** A: Mass spectrum of the imaging agent and B: Mass spectrum of albumin (M= 90582) as a reference (M= 66330). These two spectra show the full conversion of albumin into the functionalized albumin of Example 1.
**Figure 2****:** Mass spectrum of the cyanin-5-Albumine-mannose imaging agent of Example 2 (HSA-Mannose).
**Figure 3****:** Fluorescence intensity of the albumin reacted with 2 equivalents or 20 equivalents of cyanin. Figure 3 shows that less equivalents provide a higher fluorescent intensity.
**Figure 4****:** Cell Expression of E selectin. Tumor cells are CT26, Endothelial cells are Bend3 (A). In vitro evaluation of the binding (B-C) and the internalization (D) of HSA and HSA-Slx (SLX) in endothelial and tumor cells.
**Figure 5****:** Image-guided surgery: images A and B show that imaging under fluorescence (B and C) provides a higher sensitivity to image the tumoral area than white light (1). The Tumor can be resected under normal light (D), the fluorescence is put on again to evaluate residual tumor margin remaining post-surgery (E). The tumor on the side of the animal is also fluorescent.
**Figure 6****:** Biodistribution of HSA-Slx in BALB/c mice with subcutaneous colorectal CT 26 tumor.
**Figure 7****:** Accumulation of the fluorescent albumin-sialyl lewis X derivative (HSA-Slx) in CT26 colon tumors 24 hours post-injection observed by optical imaging. BG= background, Control = Albumine without biovector (HSA-CY5), sialyl refers to the optical imaging agent of Example 1 (HSA-Slx).
**Figure 8****:** Accumulation of HSA-Slx in DBA mice collagen induced arthritis model
**Figure 9****:** Distribution of HSA, HSA-Lactose (HSA-L-CY5) and HSA-Mannose (HSA-M-CY5) in the liver of balbc mice as function of time after intravenous injection.
**Figure 10****:** Distribution into monomers, dimers, trimers or oligomers of native albumin (Clinical HSA), cyanine-grafted albumin (HSA-Cyanine) and optical imaging agents of the present invention HSA-SLX and HSA-SLX2.
**Figure 11****:** *Ex vivo* imaging of extracted organs 24h after injection of HSA grafted with the tetrasaccharide Sialyl Lewix X (HSA-SLX) or HSA grafted with the trisaccharide analog of the Sialyl Lewis X of the invention described above (HSA-SLX2).
**Figure 12****:** Quantification of the *ex vivo* images with M3vision software given as the percentage of fluorescence per mice in the organs for HSA grafted with the tetrasaccharide Sialyl Lewis X (HSA-SLX) or HSA grafted with the trisaccharide analog of the sialyl Lewis X of the invention (HSA-SLX2).

### EXAMPLES

The following examples are given for illustrative purpose only.

In the following PBS stands for Phosphate Buffered Saline solution (as known in the art), and EDTA stands for ethylenediaminetetraacetic acid.

### Example 1: synthesis of an optical imaging agent according to the invention using a derivative of SLe^{x} as BIOVECTOR (compound HSA-Slx)

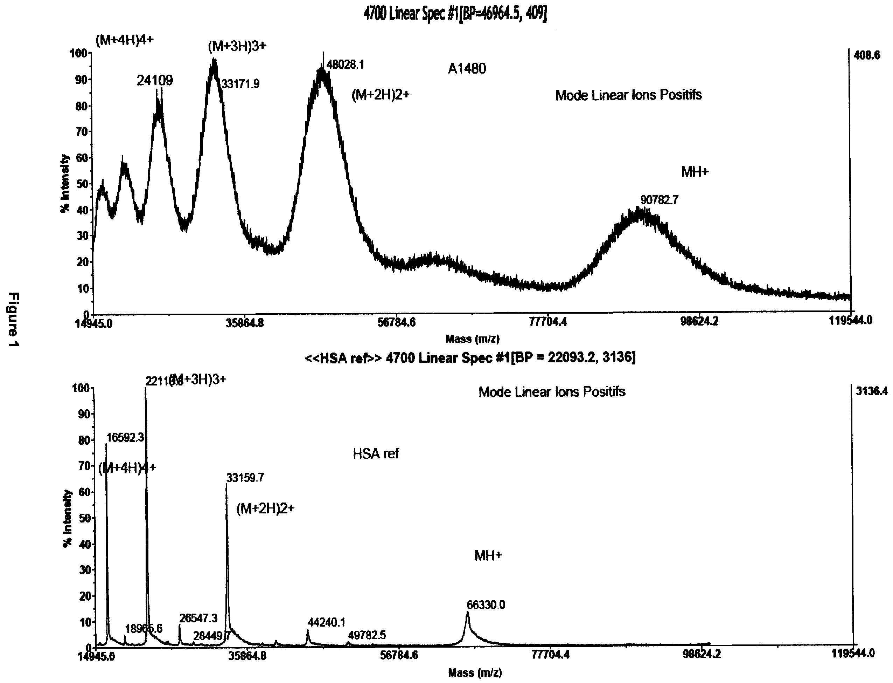

Albumin (2 mg) is coupled to N-N-disulfonate-cyanin-5-N-hydroxysuccinimide : 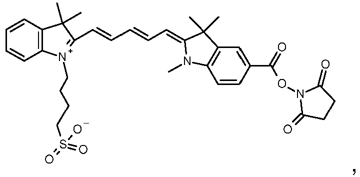 available from luminoprobe (2 equivalents per amine) in PBS and left for 30 minutes under gentle stirring. The mixture is washed with an ultrafiltration device with a 50kDa cut off at 2000 rpm during 30 minutes at 4°C. Sialyl lewis-X-maleimide is then coupled to the labelled albumin in PBS/EDTA at room temperature during 30 minutes in presence of 2-iminothiolane hydrochloride (10 equivalent per amine). A washing step is performed to remove the excess of iminothiolane and obtain the labelled targeting albumin HSA-Slx in NaCl 0,9%. The full functionalisation of albumin was confirmed by mass spectrometry with a mass of 90782 for the imaging agent (Figure 1).

Sialyl lewis-X-maleimide is obtained as follows. To Sialyl Lewis X (described in Lu et al. Carbohydrate research 383(2014) 89-96) is added of N-Succinimidyl 3-maleimidopropionate (hereafter NHS-maleimide): 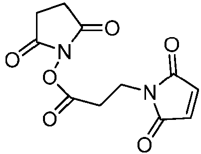 and triethylamine at room temperature, the reaction mixture is stirred for 2 hours at room temperature. The reaction mixture is then evaporated and the crude product is purified using column chromatography on silica gel.

### Example 2: synthesis of an optical imaging agent according to the invention using a derivative of mannose as BIOVECTOR (compound HSA-Mannose)

### Step 1 :

Albumin (20 mg) is coupled to N-N-disulfonate-cyanin-5-N-hydroxysuccinimide (0,1 mg) in 1 mL PBS and left for 30 minutes under gentle stirring. The mixture is washed with an ultrafiltration device with a 50kDa cut off at 2000 rpm during 30 minutes at 4°C. The intermediate compound is the cyanine-labelled albumin, which does not contain any targeting portion (no BIOVECTOR). This intermediate cyanine-labelled albumin is referred to hereinafter as HSA - CY5.

### Step 2:

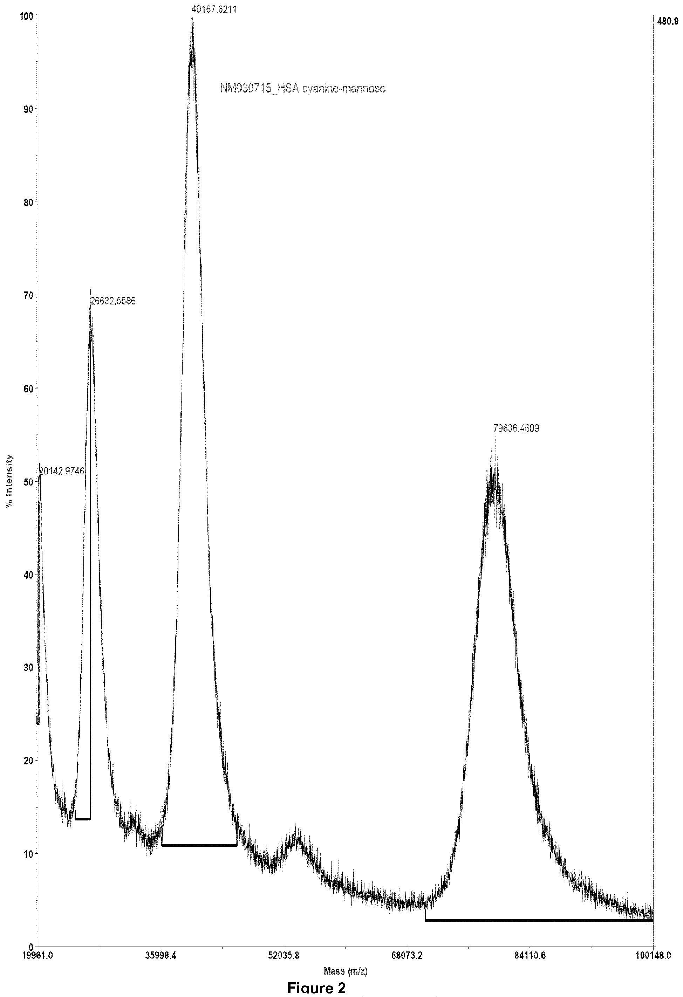

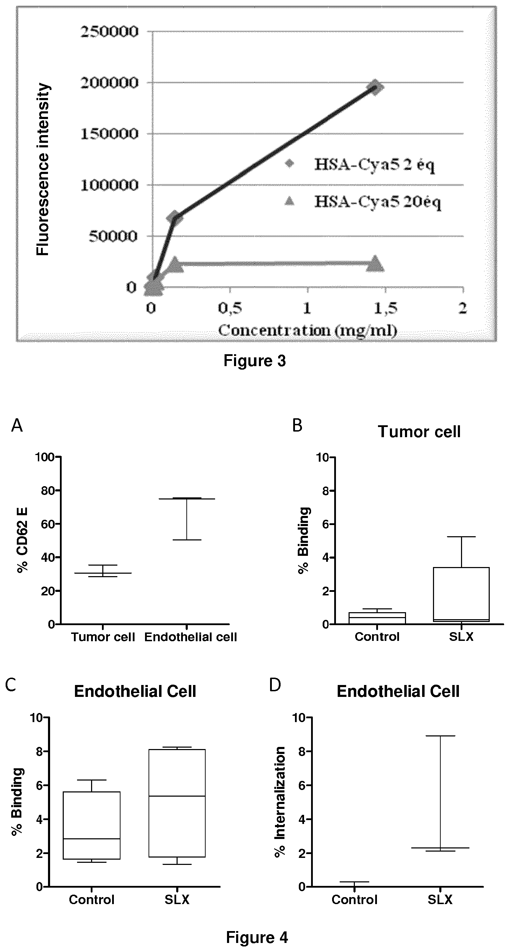

Mannose-maleimide is then coupled to the labelled albumin HSA - CY5 in PBS/EDTA at room temperature during 30 minutes in presence of 2-iminothiolane hydrochloride (10 equivalent per amine). A washing step is performed to remove the excess of iminothiolane and obtain the labelled targeting albumin in NaCl 0,9%. The expected mass of the imaging agent was confirmed by mass spectrometry (M 79636) (Figure 2). The reaction of 2 equivalents of cyanin gave the highest intensity of fluorescence as referred to 20 equivalents (Figure 3).

Mannose-maleimide 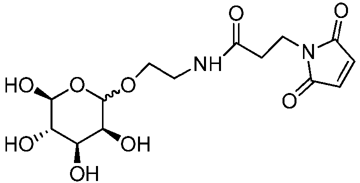 is obtained as follows. 2-aminoethylmannopyranoside 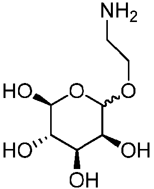 (644 mg, 2.42 mmol) is dissolved in DMF.

NHS-maleimide (491 mg, 2.2 mmol) is added, and the reaction mixture is stirred overnight. The reaction mixture is then concentrated in vacuo, and the crude product is purified using column chromatography on silica gel (eluent : dichloromethane/MeOH : 8/2). 339 mg of purified Mannose-maleimide is obtained.

### Example 3: In vitro binding affinity of the optical imaging agent of Example 1

The in vitro experiments were performed with bEnd.3 mus musculus brain endothelial cells (CRL-2299^{™}) and CT26.WT mus musculus colon carcinoma cells (CRL-2638^{™}) provided by ATCC^{®}. Those cells were cultured in DMEM-Dulbecco's Modified Eagle Medium (10566-016, Gibco Thermo Fisher) added by 10% of fetal bovine serum (10500056, Gibco Thermo Fisher) and 1% of Penicillin-Streptomycin (5,000 U/mL, 15070063, Gibco Thermo Fisher) antibiotics. A suspension of Lipopolysaccharide stimulated cells (0,1mg/mL, 4h, 37°C), were collected and prepared in binding buffer (50mM Tris HCI, 150mM NaCl, 1mM CaCl2, 1mM MgCl2, 1mM MnCl2,1% BSA in water) at 500 000 cell per milliliter. An incubation of 1h at 4°C was realized in order to saturate unspecific binding. For evaluation of E-selectin expression (Fig. 4A) cells were incubated successively with purified rat anti-Mouse CD62E antibody (1µg/mL, 550290 BD Pharmingen^{™}) in PBS/BSA 1% and with goat anti-rat IgG-Alexa Fluor ^{®}488 (1µg/ml, ab150157 Abcam) for 30min à 4°C. To study cellular binding and internalization, cells were incubated with 30ng of HSA or HSA-SLX for 20min at 4°C (Fig.4B-C) or 37°C (Fig.4D).

After those different steps, cells were washed twice with binding buffer and analyzed by flow cytometry (Guava^{®} easyCyte Millipore).

We can see that both tumor cells and endothelial cells express E-selectin. The imaging agent bind to both type of cells and is internalized mostly by endothelial cells which express E-selectin at a higher level. The non-functionalized albumin (control) neither bind, nor is internalized by endothelial cells or tumor cells.

### Example 4: Demonstration of the efficacy of the optical imaging agent of Example 1 as an aid to surgery

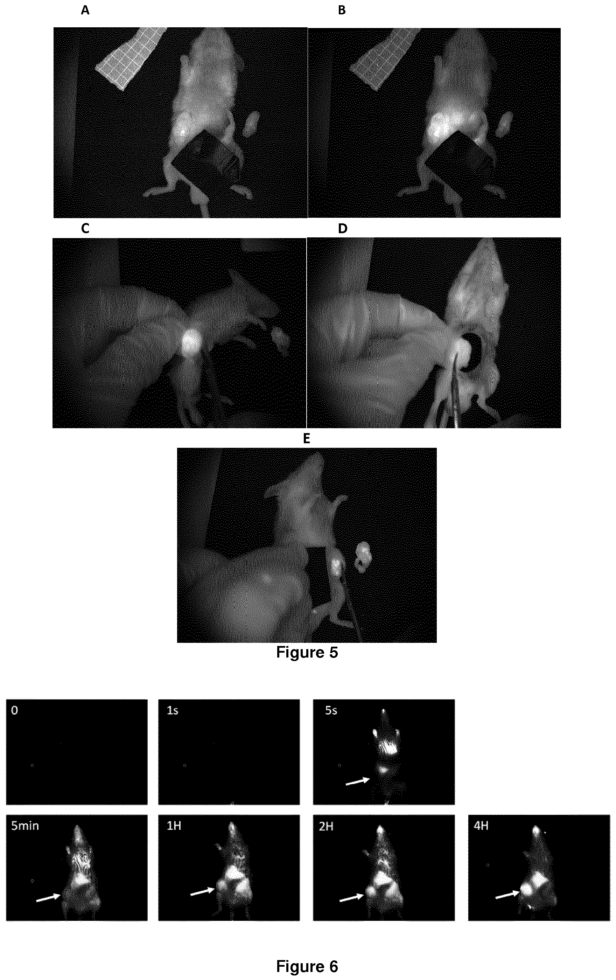

BalbC female mice (from janvier labs) were implanted with 3 mm² of CT26 tumour fragment in subcutaneous way. Fifteen day after implantation, mice were anesthetized with a mixture of ketamine (80mg/kg, Clorketam^{®} 1 000 Vetoquinol) and xylazine (10mg/kg, ROMPUN^{®} 2 % Bayer), 200µl of HSA-SLX-Cy5 (2.7mg /ml) suspension was then injected into the tail vein. Twenty four hour after injection, an evaluation of image guided surgery (Fig. 5) was realized under mice anaesthesia with Fluobeam^{®} open Imaging system for *in vivo* near infrared fluorescence imaging (λ ex 690, λ em > 700nm)λ.

### Example 5: In vivo liver accumulation of the optical imaging agent of Example 1

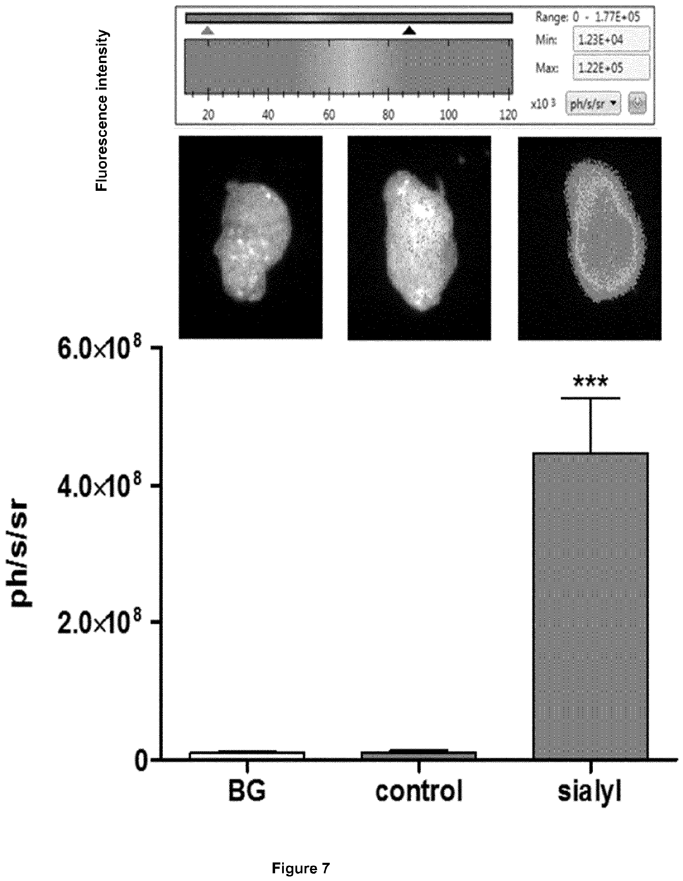

Female balbc mice (from janvier labs) were anesthetized with ketamine / xylazine mixture, 5 mice per condition were injected with HSA-CY5 and HSA-Slx suspension (2.7mg/ml) in the tail vein of Balbc mice 14 days after CT26 tumour implantation. The biodistribution kinetic of formulation was recorded by Fluobeam^{®} system as function of time (Fig.6). Twenty four hours after HSA-CY5 and HSA-SLX-CY5 injection, *ex vivo* quantification of the signal accumulated in the tumour was evaluated after blood elimination by mice PBS perfusion. Those results (Fig.7) were imaged and quantified by PhotonIMAGER^{™} optima (Biospace lab) and finally expressed in photon per second per steradian (ph/s/sr). Of note, HSA-CY5, which does not contain any targeting portion (BIOVECTOR) is used as a control.

### Example 6: In vivo paw accumulation of the optical imaging agent in collagen-induced arthritis model of Example 1

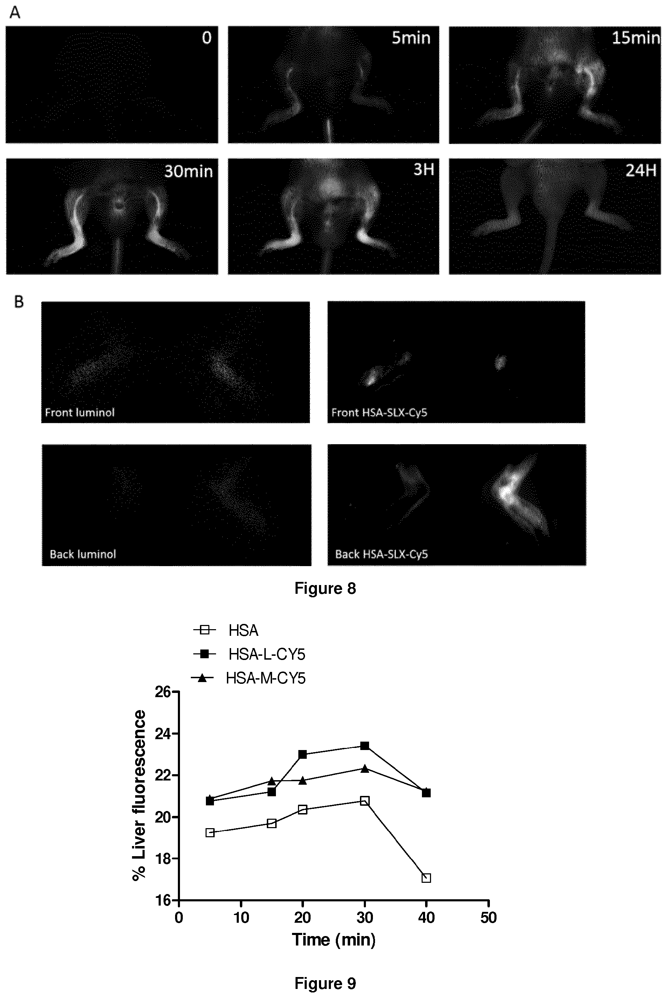

Collagen Arthritis model was induced in DBA/1 mice (from janvier labs) by injection with bovine type II collagen emulsified in Complete Freund's Adjuvant in the posterior paw articulation. Twenty four hours after arthritis induction, mice were anesthetized and injected with HSA-SLX-CY5, suspension (2.7mg/ml) in the tail vein. In vivo kinetic of the formulation was recorded by Fluobeam^{®} system at different time points (Fig. 8A). Twenty for hours after probe injection colocalization signal between probes and arthritis articulation could be evaluated by PhotonIMAGER^{™} optima (Biospace lab) after luminol injection (Fig. 8B).

### Example 7: Biodistribution and Ex vivo Quantification of the fluorescence as regard to the agent without biovector in healthy mice

Female balbc mice (from janvier labs) were anesthetized with ketamine / xylazine mixture, mice were injected with HSA-CY5, HSA-Lactose (HSA-L-CY5, obtained as described in example 1, but substituting lactose for mannose) or HSA-Mannose (HSA-M-CY5) suspension (2.7mg/ml) in the tail vein of Balbc healthy mice. In vivo liver kinetic of each formulation was recorded and quantified by the use of PhotonIMAGER^{™} optima (Biospace lab). The percent of the fluorescence detected in the liver was calculated as function of the signal of the entire mice (Fig. 9).

### Example 8: Oligomers and aggregates distributions of native albumin, cyanine grafted albumin and optical imaging agents according to the invention HSA-SLX and HSA-SLX2

Size exclusion chromatography/UV was performed to evaluate the distribution of monomers, dimers, trimers and oligomers of the optical imaging agents according to the present invention.

### Experimental:

Separation and analysis were performed on a LC-10 liquid chromatography system from Shimadzu (Kyoto, Japan) natively equipped with a vacuum degasser, an auto-sampler, a UV absorbance detector and refractive index detector (RID). The size exclusion chromatography stationary phase was a Shodex Protein LW 803 column provided by Showa Denko (Japan). Between the auto-sampler and HPLC pump, mobile phase was filtered in-line by a 0.1 µm durapore PVDF membrane in a PEEK in-line filter. Detection was realized using UV absorbance at a wavelength of 280 nm. Concomitantly, refractive index quantifications were performed using white light. Mobile phase was CH3COONH4 50 mM NaN3 0.03% in milli-Q water degassed by vacuum pump and filtered on stericup filter units. MALS detection was achieved on a three angle mini-Dawn Treos II from Wyatt Technology equipped with a 658nm wavelength laser. Data analysis was achieved on Astra 7.1, Wyatt Technology.

HSA-SLX corresponds to the optical imaging agent described in example 1.

HSA-SLX2 corresponds to the optical imaging agent described in example 1, except that the SLX moiety has been replaced by the trisaccharide of the invention with the formula below and whose synthesis is described in the above *Process of preparation* section: 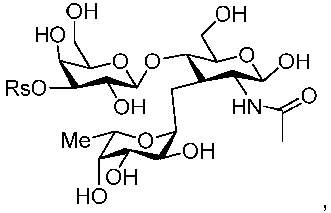 wherein Rs is SO₃Na.

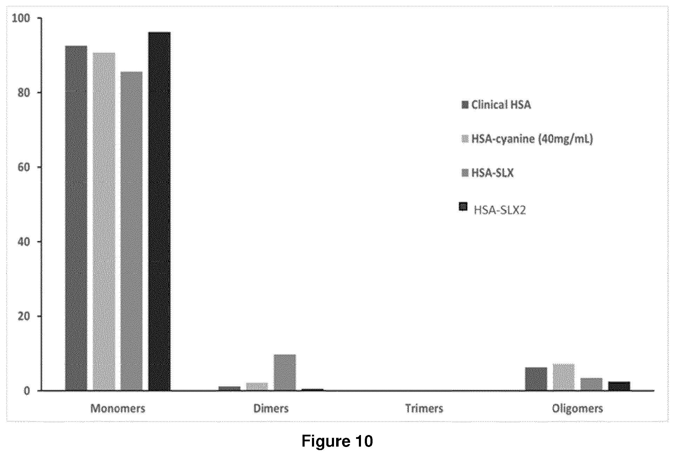

Results are illustrated on Figure 10.

The optical imaging agents according to the present invention, HSA-SLX and HSA SLX2, exist mainly in the form of monomers and form very few oligomers. Optical imaging agents according to the invention therefore barely form aggregates.

### Example 9: Biodistribution of the probe by optical imaging

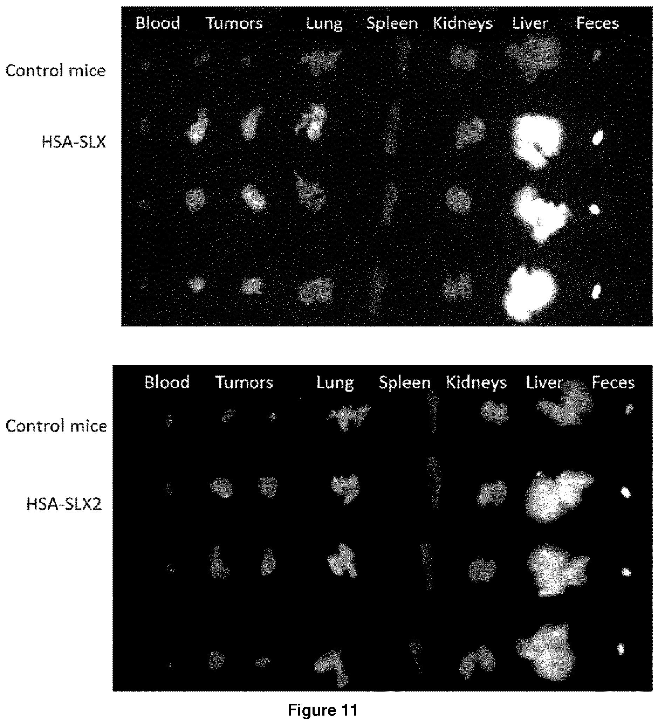

Female BALB/cJRj 8 weeks old mice (Janvier labs, Le Genest-Saint-Isle, France) were anesthetized with a mixture of 100 mg kg-1 of ketamine (IMALGENE 1000 Boehringer Ingelheim, France) and 10 mg kg-1 of xylazine (Rompun^{™}, Bayer, France) and injected by an intravenous route with 200 µl of HSA-SLX or HSA-SLX2 solutions, with both concentrations at 1 mg mL-1. Twenty four hours after the injection, the mice were sacrificed, their PBS perfused organs were placed under the camera to acquire the fluorescence signal associated with the organs (5 s, λex 640 nm, λem 780 nm). (Figure 11)

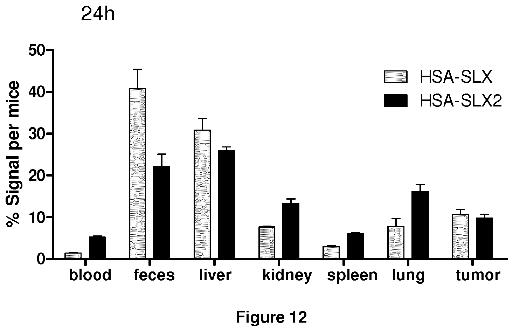

The images were processed using the M3vision software. The result is expressed as percent of the fluorescence found per mice (Figure 12).

HSA SLX and HSA SLX 2 are as described in Example 8.

Sialyl Lewis X is known in the art as the best agent to target selectin E, due to its high affinity for its target. The trisaccharide analog SLX2 of the invention appears to have the same affinity for the target than SLX and is easier to synthesize. Moreover, as shown here, grafting this analog to albumin confers similar property to the *in vivo* imaging agent, as a similar signal of fluorescence is observed with either HSA grafted with SLX or SLX2.

## Claims

1. Optical imaging agent of formula (SIGNAL)ₙ-SUPPORT-(L-BIOVECTOR)ₘ, wherein:
SUPPORT represents a physiologically acceptable chemical or biological substrate, with a particle size of between 1 and 100 nm,
SIGNAL is a fluorophore of formula (I): 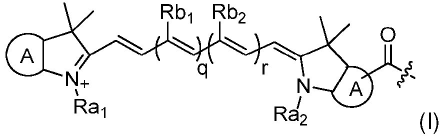 wherein
q is 0 or 1,
r is 0 or 1,
R_{b1} and R_{b2} are H or a C₁₋C₄ group, or are bridged to form a -CH₂-CH₂-CH₂- alkylene group (only when q and r represent 1),
Rₐ₁ and Rₐ₂ are identical or different and are independently a (C₁-C₆)alkyl group, optionally substituted by a SO₃⁻ group, a SO₃K group, a SO₃Na group, or a COOH group, provided that not more than one of Rₐ₁ and Rₐ₂ is substituted by a SO₃⁻ group.
Ring A represents a C₅-C₆ monocyclic aryl or heteroaryl group or a C₈-C₁₂ fused bicyclic aryl or heteroaryl group, preferably a C₅-C₆ monocyclic aryl group or a C₈-C₁₂ fused bicyclic aryl group, such as a phenyl or naphthyl group, and Ring A is optionally substituted by one SO₃⁻ or SO₃Na group, provided that not more than one of Rₐ₁ and Rₐ₂ is substituted by a SO₃⁻ group,
provided that the compound of formula (I) comprises at least one SO₃⁻ or SO₃Na group, but not more than one SO₃⁻ group, and that when the compound of formula (I) has an overall positive electric charge, it is provided as a salt, in particular a halogenide salt such as a chloride CI⁻ salt,
L is a linker of formula -C(X)-R¹-Y-, with
X being O, NH or S,
R¹ being a (C₁-C₆)alkyl group, preferably a (C₃-C₅)alkyl group, optionally interrupted by 1 to 3 groups selected from -O-, -NH-, -C(O)-, -NHC(O)-, -(O)CNH-, -C(O)-NH-N=C-, -N=C- , and 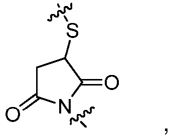
Y being NH or -(O)CNH-, and
BIOVECTOR is a carbohydrate able to target markers of inflammation of formula (II): 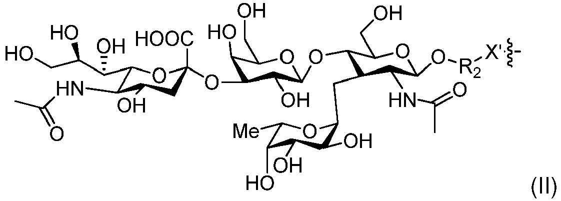
wherein R₂ represents a (C₁-C₆)alkyl group, preferably a (C₁-C₄)alkyl group,
and X' represents a heteroatom such as O, S or NH, preferably NH, or
a compound of formula (III) or (IV) or (V) or (VI) or (VII): 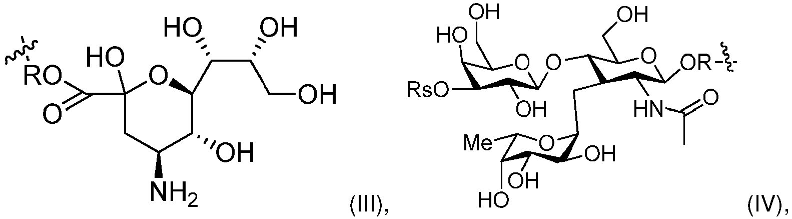 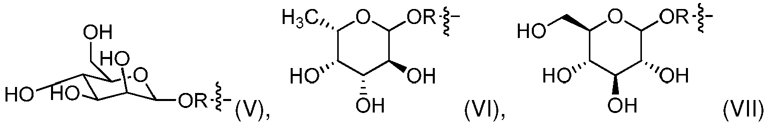
wherein R is R₂-X', with R² and X' as defined above in connection with formula (II),
and Rs is H, SO₃K or SO₃Na,,
wherein the mean grafting value n is greater than or equal to 0.5 and is less than 2,
wherein the mean grafting value m is between 1 and 30, a pharmaceutically acceptable salt, solvate or hydrate thereof.

2. The optical imaging agent of claim 1, wherein SUPPORT is a protein.

3. The optical imaging agent of claim 2, wherein SUPPORT is an albumin with a particle size of between 1 and 100 nm, advantageously a human serum albumin with a particle size of between 1 and 100 nm.

4. The optical imaging agent of any of claims 1 to 3, wherein X is NH and R¹ is a (C₁-C₆) alkyl, optionally interrupted by a 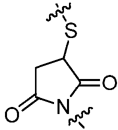 group.

5. A diagnostic composition comprising at least one optical imaging agent according to any of claims 1 to 4, a pharmaceutically acceptable salt, solvate or hydrate thereof, and at least one pharmaceutically acceptable excipient.

6. The optical imaging agent of any of claims 1 to 4 or the diagnostic composition of claim 8, for *in vivo* use as a diagnostic agent.

7. The optical imaging agent or the diagnostic composition for use of claim 6, for diagnosing diseases or conditions associated with inflammation.

8. The optical imaging agent or the diagnostic composition for use of claim 7 wherein the disease or condition associated with inflammation is selected from stroke, renal failure or cancer.

9. The optical imaging agent of any of claims 1 to 4 or the diagnostic composition of claim 5 for use in a method of surgically resecting tumours of a patient comprising the following successive steps:
- resecting the tumoral tissue identified prior to surgery in an area of interest;
- imaging the area of interest of the patient, to whom an effective amount of the optical imaging agent of any of claims 1 to 4 or diagnostic composition composition of claim 5 has been administered prior to surgery or during surgery, using an optical imaging device, so as to identify remaining tumoral tissues;
- when light signals are observed in the area of interest defining remaining tumoral tissues, proceeding with the resection of the identified remaining tumoral tissues; when no light signal is observed in the area of interest, not proceeding with any further resection in the area of interest.

10. A method of imaging a biological tissue *ex vivo,* using optical or fluorescence imaging, said biological tissue comprising the optical imaging agent of any of claims 1 to 4.

11. The method of claim 10, wherein the biological tissue is a biopsy sample.

12. A method of *ex vivo* diagnosing a disease or condition associated with inflammation, comprising the method of claim 10 or 11.

## Patentansprüche

1. Optisches Bildgebungsmittel der Formel (SIGNAL)ₙ-STÜTZE-(L-BIOVEKTOR)ₘ, wobei:
STÜTZE ein physiologisch annehmbares chemisches oder biologisches Substrat darstellt, mit einer Partikelgröße zwischen 1 und 100 nm,
SIGNAL ein Fluorophor der Formel (I) ist: 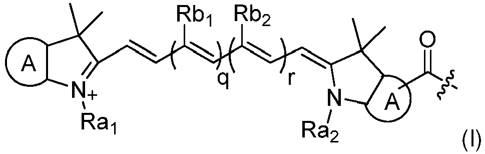 wobei
q 0 oder 1 ist,
r 0 oder 1 ist,
R_{b1} und R_{b2} H oder eine C₁-C₄-Gruppe sind oder verbrückt sind, um eine -CH₂-CH₂-CH₂-Alkylengruppe zu bilden (nur wenn q und r 1 darstellen),
Rₐ₁ und Rₐ₂ identisch oder verschieden sind und unabhängig eine (C₁-C₆)Alkylgruppe sind, optional substituiert durch eine SO₃⁻-Gruppe, eine SO₃K-Gruppe, eine SO₃Na-Gruppe oder eine COOH-Gruppe, vorausgesetzt, dass nicht mehr als eines von Rₐ₁ und Rₐ₂ durch eine SO₃⁻-Gruppe substituiert ist.
Ring A eine monocyclische C₅-C₆-Aryl- oder Heteroarylgruppe oder eine kondensierte bicyclische C₈-C₁₂-Aryl- oder Heteroarylgruppe darstellt, bevorzugt eine monocyclische C₅-C₆-Arylgruppe oder eine kondensierte bicyclische C₈-C₁₂-Arylgruppe wie eine Phenyl- oder Naphthylgruppe, und Ring A optional durch eine SO₃⁻- oder SO₃Na-Gruppe substituiert ist, vorausgesetzt, dass nicht mehr als eines von Rₐ₁ und Rₐ₂ durch eine SO₃⁻-Gruppe substituiert ist,
vorausgesetzt, dass die Verbindung der Formel (I) zumindest eine SO₃⁻- oder SO₃Na-Gruppe umfasst, aber nicht mehr als eine SO₃⁻-Gruppe, und dass, wenn die Verbindung der Formel (I) eine insgesamt positive elektrische Ladung aufweist, sie als ein Salz bereitgestellt ist, insbesondere ein Halogenidsalz wie ein Chlorid-Cl⁻-Salz,
L ein Linker der Formel -C(X)-R¹-Y- ist, wobei
X O, NH oder S ist,
R¹ eine (C₁-C₆)Alkylgruppe ist, bevorzugt eine (C₃-C₅)Alkylgruppe, optional unterbrochen durch 1 bis 3 Gruppen ausgewählt aus -O-, -NH-, -C(O)-, -NHC(O)-, -(O)CNH-, - C(O)-NH-N=C-, -N=C- und 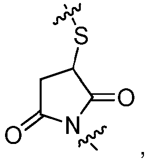
Y NH oder -(O)CNH- ist, und
BIOVEKTOR ein Kohlenhydrat ist, das in der Lage ist, Entzündungsmarker der Formel (II) zu targetieren: 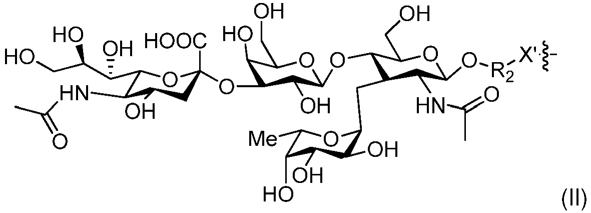
wobei R₂ eine (C₁-C₆)Alkylgruppe darstellt, bevorzugt eine (C₁-C₄)Alkylgruppe,
und X' ein Heteroatom wie O, S oder NH darstellt, bevorzugt NH, oder
eine Verbindung der Formel (III) oder (IV) oder (V) oder (VI) oder (VII): 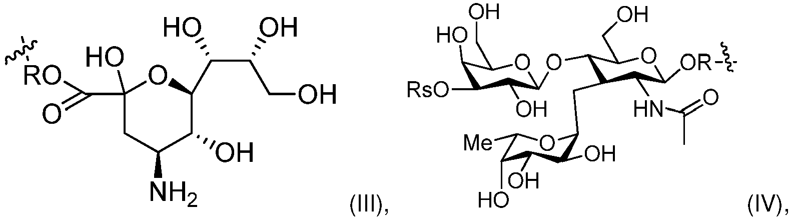 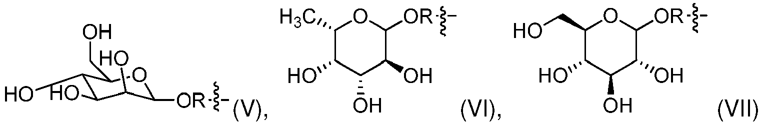
wobei R R₂-X' ist, wobei R² und X' wie oben in Verbindung mit Formel (II) definiert sind,
und Rs H, SO₃K oder SO₃Na ist,
wobei der mittlere Pfropfwert n größer als oder gleich 0,5 ist und kleiner als 2 ist,
wobei der mittlere Pfropfwert m zwischen 1 und 30, ein pharmazeutisch annehmbares Salz, Solvat oder Hydrat davon ist.

2. Optisches Bildgebungsmittel nach Anspruch 1, wobei STÜTZE ein Protein ist.

3. Optisches Bildgebungsmittel nach Anspruch 2, wobei STÜTZE ein Albumin mit einer Partikelgröße zwischen 1 und 100 nm ist, vorteilhafterweise ein menschliches Serumalbumin mit einer Partikelgröße zwischen 1 und 100 nm.

4. Optisches Bildgebungsmittel nach einem der Ansprüche 1 bis 3, wobei X NH ist und R¹ ein (C₁-C₆)Alkyl ist, optional unterbrochen durch eine 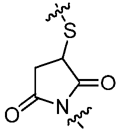 -Gruppe.

5. Diagnostische Zusammensetzung, umfassend zumindest ein optisches Bildgebungsmittel nach einem der Ansprüche 1 bis 4, ein pharmazeutisch annehmbares Salz, Solvat oder Hydrat davon und zumindest einen pharmazeutisch annehmbaren Hilfsstoff.

6. Optisches Bildgebungsmittel nach einem der Ansprüche 1 bis 4 oder diagnostische Zusammensetzung nach Anspruch 8, zur in-vivo-Verwendung als ein diagnostisches Mittel.

7. Optisches Bildgebungsmittel oder diagnostische Zusammensetzung zur Verwendung nach Anspruch 6, zum Diagnostizieren von Erkrankungen oder Zuständen assoziiert mit Entzündung.

8. Optisches Bildgebungsmittel oder diagnostische Zusammensetzung zur Verwendung nach Anspruch 7, wobei die Erkrankung oder der Zustand assoziiert mit Entzündung ausgewählt ist aus Schlaganfall, Nierenversagen oder Krebs.

9. Optisches Bildgebungsmittel nach einem der Ansprüche 1 bis 4 oder diagnostische Zusammensetzung nach Anspruch 5 zur Verwendung in einem Verfahren zum chirurgischen Resezieren von Tumoren eines Patienten, umfassend die folgenden aufeinanderfolgenden Schritte:
- Resezieren des tumorösen Gewebes, das vor Chirurgie in einem Bereich von Interesse identifiziert wird,
- Abbilden des Bereichs von Interesse des Patienten, dem eine wirksame Menge des optischen Bildgebungsmittels nach einem der Ansprüche 1 bis 4 oder der diagnostischen Zusammensetzung nach Anspruch 5 vor Chirurgie oder während Chirurgie verabreicht worden ist, unter Verwendung einer optischen Bildgebungsvorrichtung, um übriges tumoröses Gewebe zu identifizieren,
- wenn Lichtsignale in dem Bereich von Interesse beobachtet werden, die übriges tumoröses Gewebe definieren, Fortfahren mit der Resektion des identifizierten übrigen tumorösen Gewebes, wenn kein Lichtsignal in dem Bereich von Interesse beobachtet wird, kein Fortfahren mit beliebiger weiterer Resektion in dem Bereich von Interesse.

10. Verfahren zum Abbilden eines biologischen Gewebes *ex vivo,* unter Verwendung von optischer oder Fluoreszenzbildgebung, wobei das biologische Gewebe das optische Bildgebungsmittel nach einem der Ansprüche 1 bis 4 umfasst.

11. Verfahren nach Anspruch 10, wobei das biologische Gewebe eine Biopsieprobe ist.

12. Verfahren zum ex-vivo-Diagnostizieren einer Erkrankung oder eines Zustands assoziiert mit Entzündung, umfassend das Verfahren nach Anspruch 10 oder 11.

## Revendications

1. Agent d'imagerie optique de formule (SIGNAL)n-SUPPORT-(L-BIOVECTOR)m, dans laquelle :
SUPPORT représente un substrat chimique ou biologique physiologiquement acceptable, dont la taille des particules est comprise entre 1 et 100 nm,
SIGNAL est un fluorophore de formule (I) : 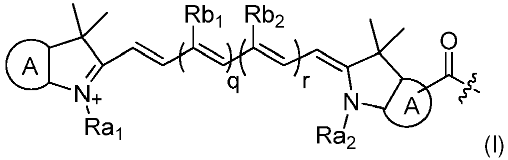 dans lequel
q est égal à 0 ou 1,
r est égal à 0 ou 1,
Rb1 et Rb2 sont H ou un groupe C1-C4, ou sont pontés pour former un groupe alkylène -CH2-CH2-CH2- (uniquement lorsque q et r représentent 1),
Ra1 et Ra2 sont identiques ou différents et sont indépendamment un groupe alkyle (C1-C6), éventuellement substitué par un groupe SO3-, un groupe SO3K, un groupe SOSNa ou un groupe COOH, à condition que pas plus d'un des groupes Ra1 et Ra2 ne soit substitué par un groupe SOS-,
l'anneau A représente un groupe aryle ou hétéroaryle monocyclique C5-C6 ou un groupe aryle ou hétéroaryle bicyclique fusionné C8-C12, de préférence un groupe aryle monocyclique C5-C6 ou un groupe aryle bicyclique fusionné C8-C12, tel qu'un groupe phényle ou naphtyle, et l'anneau A est éventuellement substitué par un groupe SO3- ou SOSNa, à condition que pas plus d'un des groupes Ra1 et Ra2 ne soit substitué par un groupe SOS-,
à condition que le composé de la formule (I) comprenne au moins un groupe SO3- ou SOSNa, mais pas plus d'un groupe SO3-, et que lorsque le composé de la formule (I) a une charge électrique positive globale, il soit fourni sous forme de sel, en particulier un sel d'halogénure tel qu'un sel de chlorure CI-,
L est un agent de liaison de formule -C(X)-R1-Y-, avec X étant O, NH ou S,
R1 est un groupe alkyle (C1-C6), de préférence un groupe alkyle (C3-C5), éventuellement interrompu par 1 à 3 groupes choisis parmi -O-, -NH-, -C(O)-, -NHC(O)-, - (O)CNH-, -C(O)-NH-N=C-, -N=C-, et 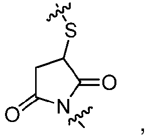
Y étant NH ou -(O)CNH-, et
BIOVECTOR est un hydrate de carbone capable de cibler les marqueurs de l'inflammation de formule (II) : 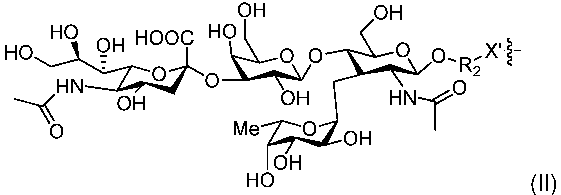
où R2 représente un groupe alkyle (C1-C6), de préférence un groupe alkyle (C1-C4),
et X' représente un hétéroatome tel que O, S ou NH, de préférence NH, ou
un composé de formule (III) ou (IV) ou (V) ou (VI) ou (VII) : 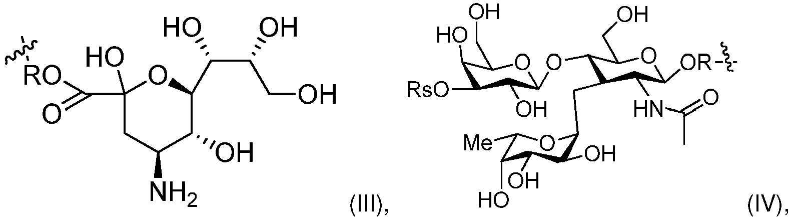 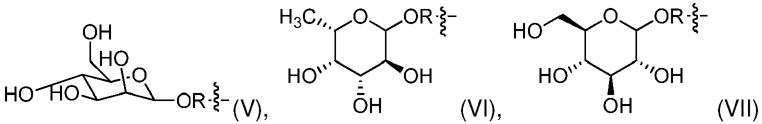
où R est R2-X', R2 et X' étant définis ci-dessus en relation avec la formule (II),
et Rs est H, SO3K ou SO3Na,
dans lequel l'indice de greffage moyen n est supérieur ou égal à 0,5 et inférieur à 2,
dans laquelle la valeur moyenne de greffage m est comprise entre 1 et 30, un sel, un solvate ou un hydrate pharmaceutiquement acceptable de celle-ci.

2. Agent d'imagerie optique selon la revendication 1, dans lequel SUPPORT est une protéine.

3. Agent d'imagerie optique selon la revendication 2, dans lequel le SUPPORT est une albumine dont la taille des particules est comprise entre 1 et 100 nm, avantageusement une sérum-albumine humaine dont la taille des particules est comprise entre 1 et 100 nm.

4. Agent d'imagerie optique selon l'une des revendications 1 à 3, dans lequel X est NH et R1 est un alkyle (C1-C6), éventuellement interrompu par un groupe

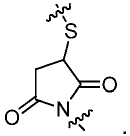

5. Composition diagnostique comprenant au moins un agent d'imagerie optique selon l'une des revendications 1 à 4, un sel, un solvate ou un hydrate pharmaceutiquement acceptable, et au moins un excipient pharmaceutiquement acceptable.

6. Agent d'imagerie optique selon l'une des revendications 1 à 4 ou composition diagnostique selon la revendication 8, pour une utilisation *in vivo* en tant qu'agent diagnostique.

7. Agent d'imagerie optique ou composition diagnostique pour son utilisation selon la revendication 6, pour diagnostiquer des maladies ou des conditions associées à l'inflammation.

8. Agent d'imagerie optique ou composition diagnostique pour son utilisation selon la revendication 7, dans laquelle la maladie ou l'état associé à l'inflammation est choisi parmi les accidents vasculaires cérébraux, l'insuffisance rénale ou le cancer.

9. Agent d'imagerie optique selon l'une des revendications 1 à 4 ou composition diagnostique selon la revendication 5 pour son utilisation dans une méthode de résection chirurgicale de tumeurs d'un patient comprenant les étapes successives suivantes :
- réséquer le tissu tumoral identifié avant la chirurgie dans une zone d'intérêt ;
- imagerie de la zone d'intérêt du patient, auquel une quantité efficace de l'agent d'imagerie optique selon l'une des revendications 1 à 4 ou de la composition diagnostique selon la revendication 5 a été administrée avant la chirurgie ou pendant la chirurgie, à l'aide d'un dispositif d'imagerie optique, afin d'identifier les tissus tumoraux restants ;
- lorsque des signaux lumineux sont observés dans la zone d'intérêt définissant les tissus tumoraux restants, procéder à la résection des tissus tumoraux restants identifiés ; lorsqu'aucun signal lumineux n'est observé dans la zone d'intérêt, ne pas procéder à une résection supplémentaire dans la zone d'intérêt.

10. Méthode d'imagerie *ex vivo* d'un tissu biologique, utilisant l'imagerie optique ou de fluorescence, ledit tissu biologique comprenant l'agent d'imagerie optique selon l'une des revendications 1 à 4.

11. Méthode selon la revendication 10, dans laquelle le tissu biologique est un échantillon de biopsie.

12. Méthode de diagnostic *ex vivo* d'une maladie ou d'un état associé à une inflammation, comprenant la méthode selon la revendication 10 ou 11.
